# EUROPEAN PATENT SPECIFICATION

(11) **EP 2 482 828 B1**
(45) Date of publication and mention of the grant of the patent: **17.12.2014**
(21) Application number: 10763945.2
(22) Date of filing: 30.09.2010
(51) Int. Cl.: A61K 35/14, A61K 35/28, A61P 13/12

(54) **HEMATOPOIETIC STEM CELLS FOR USE IN THE TREATMENT OF A KIDNEY INJURY**
HEMATOPOIETISCHE STAMMZELLEN ZUR BEHANDLUNG VON NIERENVERLETZUNGEN
CELLULES SOUCHES HÉMATOPOÏÉTIQUES POUR LEUR UTILISATION DANS LE TRAITEMENT D'UNE LÉSION RÉNALE

(30) Priority: 02.10.2009 US 248316 P
(43) Date of publication of application: 08.08.2012
(73) Proprietor: Baxter International Inc, Deerfield, IL 60015 (US); Baxter Healthcare SA, 8152 Glattpark (Opfikon) (CH)
(72) Inventor: AMRANI, David, L., Glendale WI 53209 (US); MOTLAGH, Delara, Barrington IL 60010 (US); HOFF, Catherine, M., Lake Bluff IL 60044 (US); COHEN, Amy, Grayslake IL 60030 (US); DUFFIELD, Jeremy, Seattle WA 90115 (US)
(74) Representative: Potter Clarkson LLP
(86) International application number: PCT/US2010/050812
(87) International publication number: WO 2011/041478

(56) References cited:
- WO-A2-2004/090112
- WO-A2-2007/041218
- US-A1- 2007 178 071
- LIN F ET AL: "Hematopoietic Stem Cells Contribute to the Regeneration of Renal Tubules After Renal Ischemia-Reperfusion Injury in Mice", JOURNAL OF THE AMERICAN SOCIETY OF NEPHROLOGY, WILLIAMS AND WILKINS, BALTIMORE, MD, US, vol. 14, 1 January 2003 (2003-01-01), pages 1188-1199, XP002992029, ISSN: 1046-6673, DOI: DOI:10.1097/01.ASN.0000061595.28546.A0
- PARIKH G C ET AL: "Autologous Hematopoietic Stem Cell Transplantation May Reverse Renal Failure in Patients with Multiple Myeloma", BIOLOGY OF BLOOD AND MARROW TRANSPLANTATION, KLUGE CARDEN JENNINGS PUBLISHING, CHARLOTTESVILLE, VA, US, vol. 15, no. 7, 1 July 2009 (2009-07-01), pages 812-816, XP026192842, ISSN: 1083-8791, DOI: DOI:10.1016/J.BBMT.2009.03.021 [retrieved on 2009-06-16]
- MORIGI MARINA ET AL: "The regenerative potential of stem cells in acute renal failure", CELL TRANSPLANTATION, ELSEVIER SCIENCE, US, vol. 15, no. Suppl. 1, 1 January 2006 (2006-01-01), pages S111-S117, XP009143012, ISSN: 0963-6897
- LLOYD C G: "Adult stem cells in the repair of the injured renal tubule", NATURE CLINICAL PRACTICE NEPHROLOGY, NATURE PUBLISHING GROUP, LONDON, GB, vol. 1, no. 1, 1 November 2005 (2005-11-01), page 22, XP008084383, ISSN: 1745-8323, DOI: DOI:10.1038/NCPNEPH0021
- LI BING ET AL: "Mobilized human hematopoietic stem/progenitor cells promote kidney repair after ischemia/reperfusion injury.", CIRCULATION 25 MAY 2010 LNKD- PUBMED:20458011, vol. 121, no. 20, 25 May 2010 (2010-05-25) , pages 2211-2220, XP009143032, ISSN: 1524-4539
- CARLO STELLA CARMELO ET AL: "CD34-positive cells: Biology and clinical relevance", HAEMATOLOGICA, vol. 80, no. 4, 1995, pages 367-387, ISSN: 0390-6078

## Description

### BACKGROUND

Although the kidney has tremendous capacity for regeneration, chronic kidney disease and kidney failure following acute kidney injury or following both repetitive and chronic kidney injuries are now leading causes of morbidity and mortality in the world [1-3]. Furthermore chronic kidney disease has been identified as a leading independent risk factor for cardiovascular diseases and cardiovascular mortality [4]. Chronic kidney diseases may represent unsuccessful or inadequate renal repair following injury, and currently there are few therapies that promote repair and regeneration of the kidney [5].

The kidney peritubular microvasculature has received increasing attention recently, since this fragile vasculature may not regenerate normally following injury. This may predispose to chronic ischemia of the kidney [12-15], triggering chronic inflammation, tubular atrophy, and interstitial fibrosis, hallmarks of chronic kidney disease [12, 13]. It has been proposed that unsuccessful regeneration of peritubular capillaries following injury is central to progression to chronic kidney diseases [12-14].

There has been much interest in the reparative and angiogenic properties of stem cells from bone marrow [6-8], and several studies in mouse models of kidney disease have shown that mouse mesenchymal stromal cells of bone marrow (MSCs) can prevent or attenuate kidney injury, possibly by paracrine or systemic secretory mechanisms [6, 9, 10]. However the possible angiogenic role of hematopoietic stem cells (HSCs) in kidney repair has been little explored and no studies have ascertained the practicability of harvesting human HSCs in cell therapy to promote organ repair and regeneration [11].

Thus there exists a need in the art to develop methods for the treatment of a kidney injury.

### BRIEF SUMMARY OF THE INVENTION

The studies described herein demonstrate for the first time that human CD34+ stem cells are recruited to the injured kidney and promote survival, vascular regeneration and functional recovery. The capacity of human CD34+ hematopoietic stem cells to promote repair and regeneration of the kidney was studied using an established ischemia reperfusion injury model in mice. Human HSCs administered, e.g., systemically following kidney injury, were selectively recruited to injured kidneys of the mice and localized prominently in and around vasculature. This recruitment was associated with enhanced repair of the kidney microvasculature, tubule epithelial cells, enhanced functional recovery and increased survival. In some instances, HSCs acquired early myeloid and lymphoid differentiation markers in the kidney and also showed acquisition of endothelial progenitor cell markers, but retained synthesis of high levels of pro-angiogenic transcripts following recruitment to the kidney. Although infused purified HSCs contained small numbers of circulating endothelial progenitors and occasional endothelial cells, only rare human endothelial cells were identified in the mouse capillary walls, suggesting HSC-mediated renal repair is by paracrine mechanisms rather than replacement of vasculature. These studies advance human HSCs as a promising therapeutic strategy for promoting renal repair following injury.

Further provided herein are related methods comprising the administration of HSCs. For example, a method of preventing a renal disease or renal medical condition in a patient comprising a kidney injury, a method of increasing survival of a patient comprising a kidney injury, and a method of preventing a non-renal disease or non-renal medical condition which is caused by or associated with a renal disease or renal medical condition in a patient comprising a kidney injury.

In some embodiments disclosed herein, the HSCs are formulated with a pharmaceutically acceptable carrier. Accordingly, a pharmaceutical composition comprising a population of HSCs and a pharmaceutically acceptable carrier is disclosed. In certain embodiments, the pharmaceutical composition comprises additional therapeutic agents or diagnostic agents, optionally, conjugated to the HSCs. Such pharmaceutical compositions can be used to deliver the therapeutic agent or diagnostic agent to an injured kidney. Therefore, HSCs for use as a therapeutic agent or a diagnostic agent to an injured kidney in a patient, comprising administering to the patient HSCs conjugated to the therapeutic agent or diagnostic agent, are disclosed.

The pharmaceutical composition comprises a heterogeneous population of cells, wherein the CD34+ HSCs constitute at least 70%, at least about 80%, at least about 90%, at least about 95%, or at least about 98% of the cells of the population. Further embodiments of the pharmaceutical compositions and uses thereof are provided herein.

### BRIEF DESCRIPTION OF THE DRAWINGS

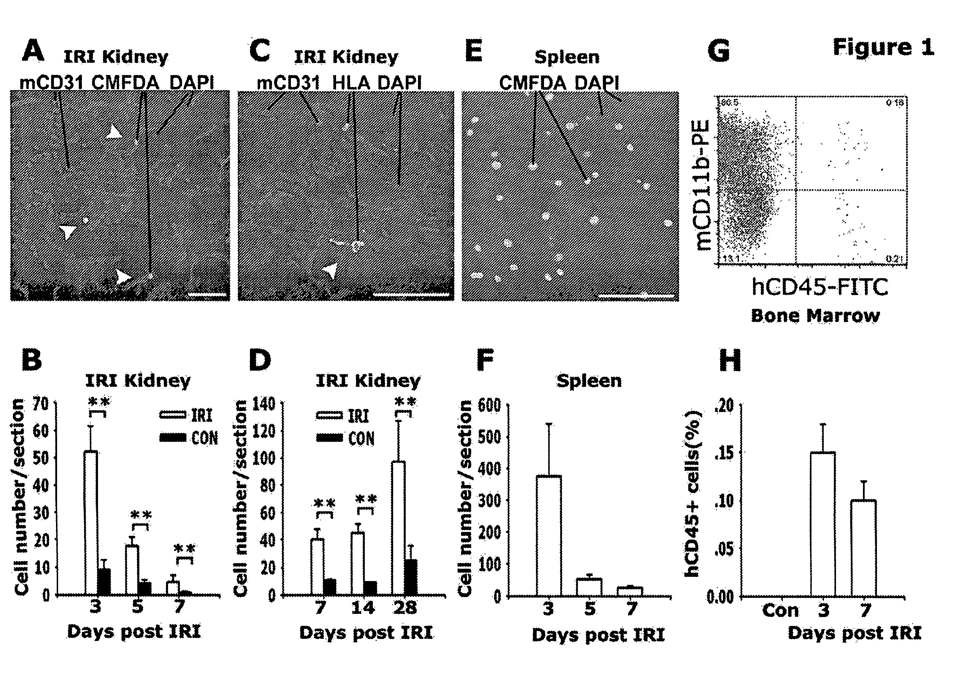

Figure 1 demonstrates that human hematopoietic stem cells are recruited to post ischemia reperfusion injury kidneys, spleen and bone marrow of NOD/SCID mice. (A) Representative confocal image of day 3 post IRI kidney of NOD/SCID mice that received adoptively transferred human HSCs on d1 post IRI showing CMFDA labeled human cells (arrows) in peritubular capillaries denoted by mouse CD31 labeling. (B) Graph indicating the number of human HSCs identified in post IRI and control kidneys on days 3, 5 and 7 after IRI. (C) Representative confocal image detecting human HLA class I (green, arrow) of day 7 post IRI kidney of NOD/SCID mice treated with human HSCs 24h after IRI. (D) Graph indicating the number of human HLA class I cells per section in post IRI and control kidneys on days 7, 14 and 28 after IRI. (E) Representative confocal image of CMFDA labeled human HSCs in spleen 3d following IRI, adoptively transferred 1d after kidney IRI. (F) Graph indicating the number of CMFDA positive cells per section in the spleen on days 3, 5 and 7 after IRI. (G) Representative flow cytometric plot for CD11b (detects mouse and human antigens) and human CD45 of whole bone marrow from d7 post kidney IRI mouse that received adoptively transferred human HSCs d1 after IRI. (H) Graph indicating proportion of human CD45+ cells in mouse bone HSCs (E) on d1 and d2. Note prominent debris in severely injured tubules in (D), present to a much lower extent in (E). (F) Graph showing tubular injury index for mice following vehicle or HSCs (n=3 per timepoint). (G-H) Representative images of Sirius red-stained kidneys d28 post IRI that received either vehicle (G) or HSCs (H) on d1 and d2 post IRI. (I) Graph showing fibrosis area for mice following vehicle or HSCs (n=3 per timepoint). Mean ± SD. **P* < 0.05, vs. vehicle group. (Bars = 50µm).

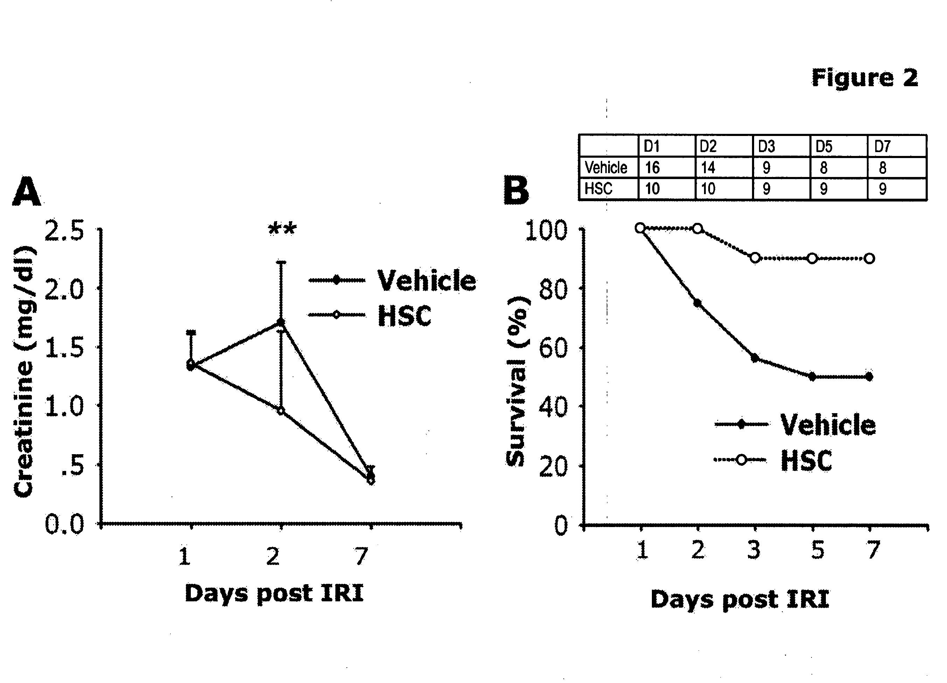

Figure 2 demonstrates that adoptive transfer of human HSCs to NOD/SCID mice following kidney ischemia reperfusion injury decreases mortality and improves kidney function. (A) Plasma creatinine levels on days 1, 2 and 7 following bilateral IRI followed IV injection with PBS (Vehicle, n=16) or 2.5X10⁶ (HSC, n=10) 1day following injury. Data are mean ± SD. P value <0.01. (B) Survival curves and number at each time point, for mice undergoing bilateral IRI followed IV injection with PBS (vehicle) or 2.5X10⁶ human HSCs (HSC) 1 day following injury. P value = 0.039.

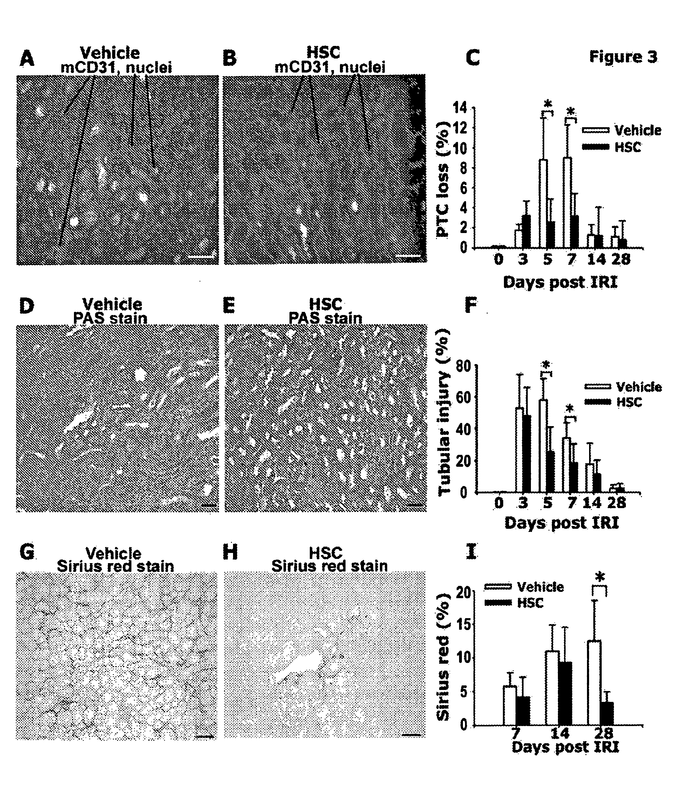

Figure 3 demonstrates that adoptive transfer of Human HSCs attenuates peritubular capillary loss and reduces tubular epithelial injury following kidney ischemia reperfusion injury. (A-B) Representative images of mouse CD31-labeled peritubular capillaries (PTC) of outer medulla of d7 post IRI kidney that received vehicle (A) or HSCs (B) on d1 and d2. Note marked PTC loss in (A). (C) Graph showing PTC index for mice following vehicle or HSCs (n=3 per timepoint). (D-E) Representative light images of PAS stained kidney sections of outer medulla d5 post IRI kidney from mice that received vehicle (D) or HSCs (E) on d1 and d2. Note prominent debris in severely injured tubules in (D), present to a much lower extent in (E). (F) Graph showing tubular injury index for mice following vehicle or HSCs (n=3 per timepoint). (G-H) Representative images of Sirius red-stained kidneys d28 post IRI that received either vehicle (G) or HSCs (H) on d1 and d2 post IRI. (I) Graph showing fibrosis area for mice following vehicle or HSCs (n=3 per timepoint). Mean ±SD. **P* <0.05, vs. vehicle group. (Bars = 50µm).

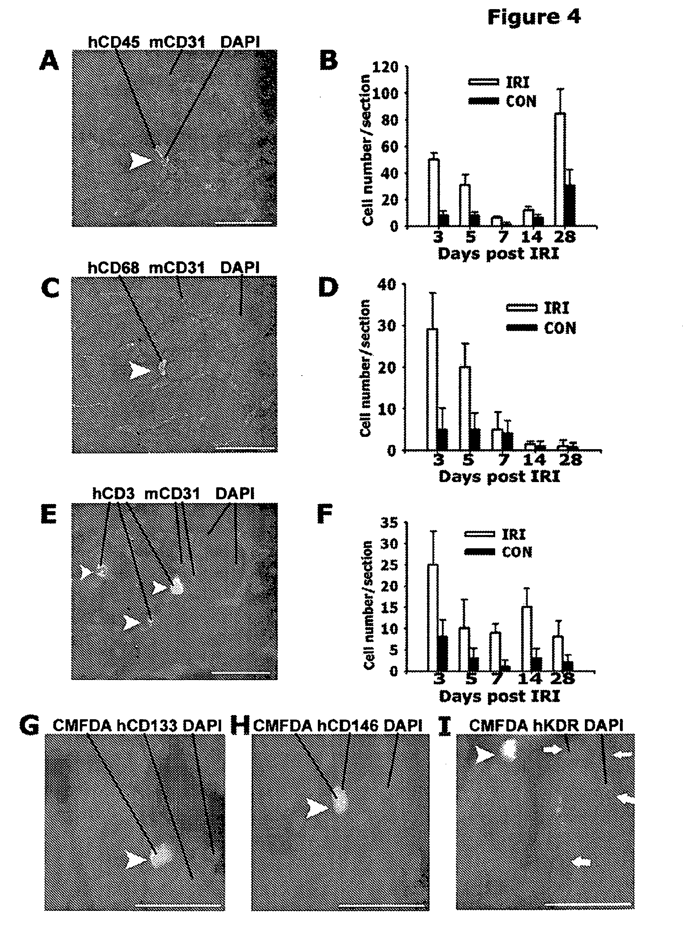

Figure 4 demonstrates the differentiation of human HSCs in kidneys. Representative confocal images (A, C) and epifuorescence image (E) of kidney outer medulla showing expression of human CD45 (A), human CD68 (C), and human CD3 (E) in cells (arrowheads) in d5 post IRI kidneys that received IV injection of HSCs 1 day following injury, colabeled to show mCD31 (red) of the mouse vasculature. Graphs showing the number of human CD45 (B), human CD68 (D), and human CD3 (F) cells identified in post IRI kidneys and control kidneys. Data are mean ± SD. n = 6/ timepoint. (G-H) Representative epifluorescence images of day 3 post IRI kidney of NOD/SCID mice that received human HSCs on d1-d2 labeled with CMFDA (arrowheads) co-labeled with antibodies against human CD 133 (G), CD 146 (H) and KDR (I). Note anti-KDR antibodies also detected mouse endothelium (arrows) (Bars = 50µm).

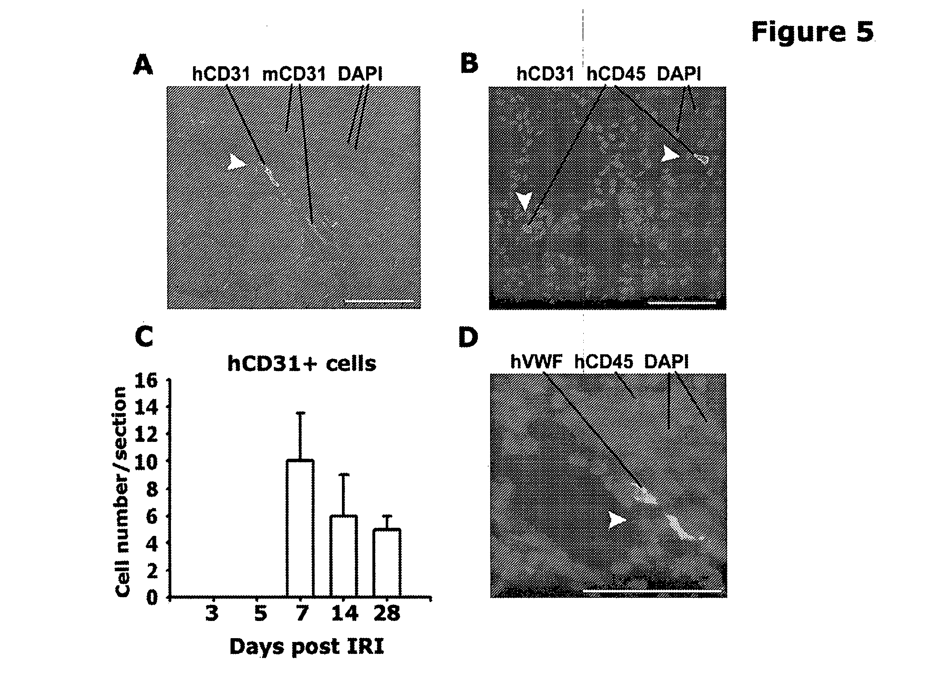

Figure 5 demonstrates that rare human endothelial cells can be detected in the kidney after ischemic injury and HSC infusion. (A-B) Confocal images of d28 post IRI kidneys showing the presence of human CD31 expressing cells some of which appear to be integrated into capillaries (arrowhead) (A) but the majority are morphologically monocytic and co-express hCD45 (arrowheads) (B). (C) Graph showing the number of human CD31 expressing cells in the post IRI kidneys with time following adoptive transfer of HSCs 1day following injury. (D) Specific expression of human von Willebrand factor (vWF) (arrowheads) and not mouse vWF in cells that lack expression of human CD45 in the post IRI kidney (Bar = 50µm).

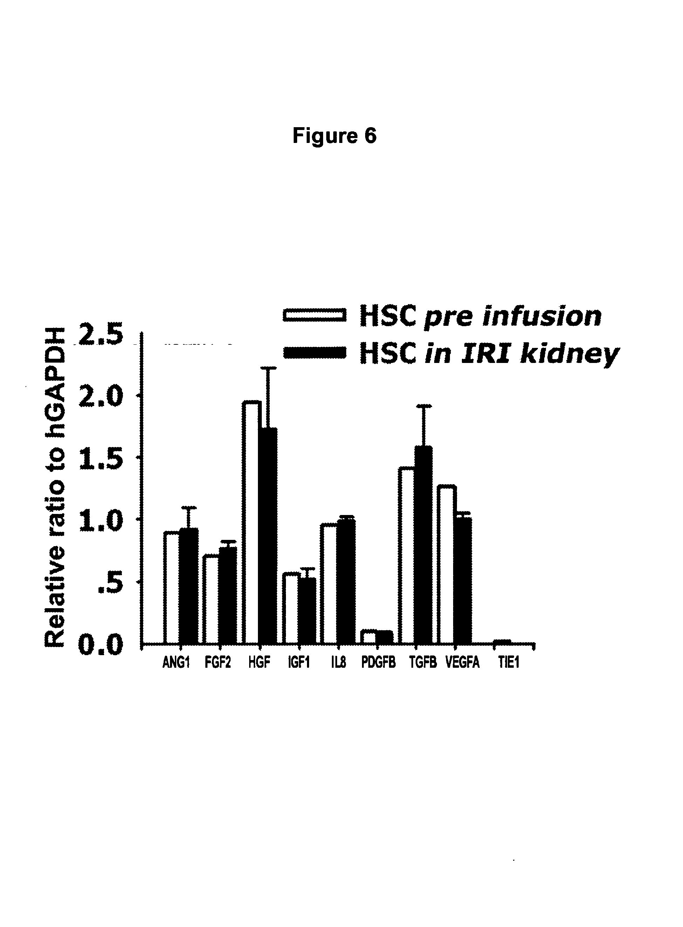

Figure 6 demonstrates human HSCs generate angiogenic paracrine factors in the kidney after kidney ischemic injury. Relative gene expression compared with GAPDH of pro-angiogenic transcripts in HSCs (white) and HSCs prior to adoptive transfer and, purified from post IRI kidney 48h following adoptive transfer. Note that HSCs recruited to the kidney retain high-level expression of pro-angiogenic transcripts. Mean ± SD. n = 6/group. (Bars = 50µm).

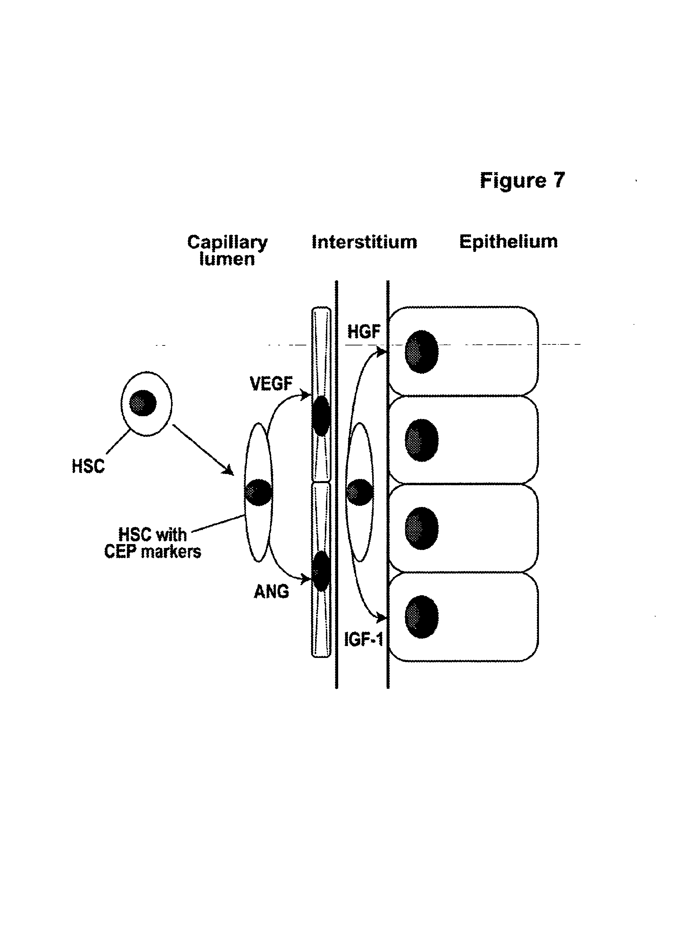

Figure 7 demonstrates a model of functions of HSCs in repair of the kidney following injury. HSCs are recruited to the injured kidney where they acquire the CEP marker CD146 and localize within injured capillaries and in the interstitium. Local production of cytokines including Angiopoietins, Vascular endothelial growth factors, haptocyte growth factor and insulin like growth factors are generated promoting cellular repair by paracrine mechanisms.

### DETAILED DESCRIPTION OF THE INVENTION

Data described herein demonstrate for the first time the specific localization of administered HSCs to a mammal with an injured kidney and subsequent repair of the injured kidney tissues, including, but not limited to the peritubular microvasculature, which repair is mediated by the HSCs. The disclosure accordingly provides HSCs for use in treating a kidney injury in a patient, wherein the HSCs are administered to the patient in an amount effective to treat the kidney injury in the patient. The invention is directed to an amount of hematopoietic stem cells (HSCs) for use in treating a kidney injury in a patient or preventing a renal disease or renal medical condition in a patient comprising a kidney injury, or increasing survival of a patient comprising a kidney injury, wherein the amount of HSCs is to be administered at least 20 hours post injury and before 7 days post injury, wherein the amount is effective to treat the kidney injury in the patient, to prevent the renal disease or renal medical condition in the patient, or to increase survival of the patient respectively; and wherein the HSCs are part of a heterogeneous cell population of which at least 70% of the population are CD 34+ cells.

The term "treat," as well as words stemming therefrom, as used herein, does not necessarily imply 100% or complete amelioration of a targeted condition. Rather, there are varying degrees of a therapeutic effect which one of ordinary skill in the art recognizes as having a benefit. In this respect, the methods described herein provide any amount or any level of therapeutic benefit of a kidney injury and therefore "treat" the injury. For example, in various aspects, the method of treating a kidney injury includes one or more of: promoting repair or regeneration of the injured kidney tissue of the patient, increasing survival of the patient, enhancing functional recovery of the kidney, or restoring function to the kidney. In another aspect, the treatment provided by the method includes amelioration of one or more conditions or symptoms caused by the injured kidney. By way of example and without limitation, the methods described herein achieve one or more of the following: enhanced repair or regeneration of the kidney peritubular microvasculature (e.g., the peritubular capillaries), creation or stabilization of blood vessels (e.g., peritubular microvasculature (e.g., the peritubular capillaries)) in the kidney, inducement of angiogenesis in the injured kidney, or enhanced repair of the tubule epithelial cells, reducing the occurrence of negative remodeling of the kidney.

### Kidney Injuries

In various aspect of the disclosure, the HSCs provided are intended to treat kidney injury in the patient which is any injury to the kidney caused by any one or more of: ischemia, exposure to a toxin, use of an angiotensin-converting enzyme inhibitor (ACEI) or angiotensin II receptor blocker, a blood transfusion reaction, an injury or trauma to muscle, surgery, shock, hypotension, or any of the causes of ARF or chronic kidney disease, as further described herein.

The targeted kidney injury comprises injury to any tissue found within the kidney, including, but not limited to, a tissue of the medulla, cortex, renal pyramid, interlobar artery, renal artery, renal vein, renal hilum, renal pelvis, ureter, minor calyx, renal capsule, inferior renal capsule, superior renal capsule, interlobar vein, nephron, major calyx, renal papilla, glomerulus, Bowman's capsule, and renal column, which tissue is sufficiently damaged to result in a partial or complete loss of function. The injured kidney tissue comprises any one or more of distinct cell types which occur in the kidney, including, but not limited to, kidney glomerulus parietal cells, kidney glomerulus podocytes, intraglomerular mesangial cells, endothelial cells of the glomerulus, kidney proximal tubule brush border cells, loop of Henle thin segment cells, thick ascending limb cells, kidney distal tubule cells, kidney collecting duct cells, and interstitial kidney cells. In certain embodiments of the invention, the kidney injury comprises injury to a kidney peritubular microvasculature. In certain aspects, the kidney injury comprises injury to a peritubular capillary. In some embodiments, the kidney injury comprises injury to tubule (tubular) epithelial cells.

### Prevention of Renal disease and Renal Medical Conditions

While the kidney has tremendous capacity for self-repair or self-regeneration, a kidney injury often leads to an increased predisposition to a renal disease or renal medical condition. It is theorized that the HSCs for treating a kidney injury in a patient provided herein allow for successful repair and regeneration of the kidney, so that the patient does not have an increased predisposition to a renal disease or renal medical condition. Therefore, the disclosure further provides HSCs for use in preventing a renal disease or renal medical condition in a patient comprising a kidney injury. The HSCs are administered to the patient in an amount effective to prevent the renal disease or renal medical condition. In some embodiments described herein, the amount is effective to treat the kidney injury, e.g., an amount effective to restore kidney function, to regenerate kidney peritubular microvasculature.

As used herein, the term "prevent" as well as words stemming therefrom, does not necessarily imply 100% or complete prevention. Rather, there are varying degrees of prevention of which one of ordinary skill in the art recognizes as having a potential benefit. In this respect, the methods of preventing described herein provide any amount or any level of prevention of renal disease or renal medical condition. In various aspects, the method of preventing is a method of delaying, slowing, reducing, or attenuating the onset, development, occurrence, or progression of the renal disease or renal medical condition, or a symptom or condition thereof.

In some embodiments described herein, the renal disease or renal medical condition prevented is acute renal failure, chronic kidney disease, renal interstitial fibrosis, diabetic nephopathy, glomerulonephritis, hydronephrosis, interstitial nephritis, kidney stones (nephrolithiasis), kidney tumors (e.g., Wilms tumor, renal cell carcinoma), lupus nephritis, minimal change disease, nephrotic syndhrome, pyelonephritis, renal failure (e.g., other than acute renal failure and chronic kidney disease).

### Acute Renal Failure

The term "acute renal failure" as used herein is synonymous with "acute kidney injury" or "ARF" and refers to a rapid loss of renal function due to damage to the kidneys. ARF is a complex syndrome marked by abrupt changes in the levels of nitrogenous (e.g., serum creatine and/or urine output) and non-nitrogenous waste products that are normally excreted by the kidney. The symptoms and diagnosis of ARF are known in the art. See, for example, Acute Kidney Injury, Contributions to Nephrology, Vol. 156, vol. eds. Ronco et al., Karger Publishers, Basel, Switzerland, 2007, and Bellomo et al., Crit Care 8(4): R204-R212, 2004.

In various aspects, the ARF is a pre-renal ARF, an intrinsic ARF, or a post-renal ARF, depending on the cause. In this regard, the pre-renal ARF may be caused by one or more of: hypovolemia (e.g., due to shock, dehydration, fluid loss, or excessive diruretic use), hepatorenal syndrome, vascular problems (e.g., atheroembolic disease, renal vein thrombosis, relating to nephrotic syndrome), infection (e.g., sepsis), severe burns, sequestration (e.g., due to pericarditis, pancreatitis), and hypotension (e.g., due to antihypertensiveness, vasodilator use).

The intrinsic ARF may be caused by one or more of: toxins or medications (e.g., NSAIDs, aminoglycoside antibiotics, iodinated contrast, lithium, phosphate nephropathy (e.g., associated with colonoscopy bowel preparation with sodium phosphates), rhabdomyolysis (e.g., caused by injury (e.g., crush injury or extensive blunt trauma), statins, stimulant use), hemolysis, multiple myeloma, acute glomerulonephritis.

The post-renal ARF may be caused by one or more of: medication (e.g., anticholinergics), benign prostatic hypertrophy or prostate cancer, kidney stones, abdominal malignancy (e.g., ovarian cancer, colorectal cancer), obstructed urinary catheter, and drugs that cause crystalluria or myoglobulinuria, or cystitis.

ARF may be caused by ischemia, a toxin, use of an angiotensin-converting enzyme inhibitor (ACEI) or angiotensin II receptor blocker, a blood transfusion reaction, an injury or trauma to muscle, surgery, shock, and hypotension in the patient. The toxin which causes ARF can be an antifungal or a radiographic dye. Also, in some embodiments, ARF involves acute tubular necrosis or renal ischemia reperfusion injury.

### Chronic Kidney Disease

As disclosed herein, the HSCs are for use in preventing a renal disease or renal medical condition, the renal disease is chronic kidney disease (CKD). As used herein, "chronic kidney disease," which is also known as "chronic renal disease," refers to a progressive loss of renal function over a period of months or years. The CKD being treated is any stage, including, for example, Stage 1, Stage 2, Stage 3, Stage 4, or Stage 5 (also known as established CKD, end-stage renal disease (ESRD), chronic kidney failure (CKF), or chronic renal failure (CRF)).

The CKD may be caused by any one of a number of factors, including, but not limited to, acute kidney injury, causes of acute kidney injury, Type 1 and Type 2 diabetes mellitus leading to diabetic nephropathy, high blood pressure (hypertension), glomerulonephritis (inflammation and damage of the filtration system of the kidneys), polycystic kidney disease, use (e.g., regular and over long durations of time) of analgesics (e.g., acetaminophen, ibuprofen) leading to analgesic nephropathy, atherosclerosis leading to ischemic nephropathy, obstruction of the flow of urine by stones, an enlarged prostate, strictures (narrowings), HIV infection, sickle cell disease, heroin abuse, amyloidosis, kidney stones, chronic kidney infections, and certain cancers.

### Prevention of Non-Renal Diseases and Non-Renal Medical Conditions

Chronic kidney disease has been identified as a leading independent risk factor for cardiovascular diseases and cardiovascular mortality. It is theorized that the administration of HSCs as described herein is furthermore useful for preventing diseases or medical conditions other than renal diseases and renal medical conditions. Accordingly, HSCs for use in preventing a non-renal disease or non-renal medical condition which is caused by or associated with a renal disease or renal medical condition in a patient comprising a kidney injury is further provided herein. The HSCs are administered to the patient in an amount effective to prevent the non-renal disease or non-renal medical condition. In certain embodiments, the non-renal disease or non-renal medical condition is cardiovascular disease.

### Increasing Survival

Chronic kidney disease and kidney failure following acute kidney injury or following both repetitive and chronic kidney injuries are now leading causes of morbidity and mortality in the world. It is theorized herein that the administration of HSCs as described herein is furthermore useful for preventing mortality (increasing survival) of a patient comprising a kidney injury. Accordingly, HSCs for use in increasing survival of a patient comprising a kidney injury is furthermore provided herein. The HSCs are administered to the patient in an amount effective to increase survival of the patient.

### Hematopoietic Stem Cells (HSCs)

For purposes herein, the term "hematopoietic stem cells" or "HSCs" refer to multipotent stem cells that give rise to the blood cell types, including, for example, myeloid (monocytes and macrophages, neutrophils, basophils, eosinophils, erythrocytes, megakaryocytes, platelets, dendritic cells), and lymphoid lineages (T-cells, B-cells, natural killer (NK) cells). The HSCs may be multipotent, oligopotent, or unipotent HSCs.

### Methods of Obtaining HSCs and Sources of HSCs

The HSCs may be obtained by any means known in the art. In some embodiments, the HSCs are isolated from a donor. The term "isolated" as used herein means having been removed from its natural environment. The HSCs are isolated from any adult, fetal or embryonic tissue comprising HSCs, including in various aspects, but not limited to, bone marrow, adipose tissue, blood, yolk sac, myeloid tissue (e.g., in the liver, spleen, in fetuses, e.g., fetal liver, fetal spleen), umbilical cord blood, placenta, and aorta-gonad-mesonephros.

The donor of the HSCs is any of the hosts described herein with regard to patients. In some aspects, the donor is a mammal. In specific aspects, the donor is a human. In other aspects, the donor of HSCs is the same as the patient. In this regard, the HSCs are considered "autologous" to the patient. In some embodiments, the donor of the HSCs is different from the patient, but the donor and patient are of the same species. In this regard, the HSCs are considered as "allogeneic."

In some embodiments, the HSCs are isolated from bone marrow of a donor, e.g., the hip of a donor, using a syringe and needle. In other embodiments, HSCs are isolated from the blood (e.g., peripheral blood). In certain aspects, the HSCs are isolated from the blood following pre-treatment of the donor with cytokines which induce or promote mobilization of the HSCs from the bone marrow into the blood, e.g., peripheral blood. The cytokine in some instances is granulocyte-colony stimulating factor (G-CSF), granulocyte macrophage colony stimulating factor (GM-CSF), or AMD-3100.

In some embodiments, the HSCs are primary cells or freshly isolated cells. Alternatively, the HSCs are cultured cells, cells of an established cell line, and/or thawed from frozen stocks of HSCs. HSCs can be obtained through cell repositories, such as, for example, the American Tissue Culture Collection (ATCC), the National Stem Cell Resource (NSCR), National Stem Cell Bank (NSCB), as well as other commercial vendors.

In other aspects, further steps to obtain a particular population of HSCs are performed. The HSCs in some embodiments are purified. The term "purified" as used herein means having been increased in purity, wherein "purity" is a relative term, and not to be necessarily construed as absolute purity. In various aspect, for example, the purity is at least about 50%, can be greater than 60%, 70% or 80%, or can be 100%. Accordingly, in some embodiments the HSCs of the invention are part of a heterogenous population of cells or part of a substantially homogenous population of cells. For example, in some aspects the HSCs are a clonal population of HSCs, wherein each cell of the population is genetically indistinct from another cell of the population. The heterogeneous population of cells comprise other types of cells, cells other than HSCs. For example, in some aspects the heterogeneous population of cells comprise, in addition to the HSCs, a white blood cells (a white blood cells of myeloid lineage or lymphoid lineage), a red blood cell, an endothelial cell, circulating endothelial precursor cells, an epithelial cell, a kidney cell, a lung cell, an osteocyte, a myelocyte, a neuron, smooth muscle cells. Alternatively or additionally, the heterogeneous population of cells comprises only HSCs, but the HSCs are not clonal, e.g., not genetically indistinct from each other. The HSCs of the heterogeneous population have different phenotypes as discussed further herein. Suitable methods of isolating cells, e.g., HSCs, having a particular phenotype are known in the art and include, for instance, methods using optical flow sorters (e.g., fluorescence-activated cell sorting (FACS)) and methods using non-optical flow sorters (e.g., magnetic-activated cell sorting).

### Markers Expressed by HSCs

The HSCs described herein have any phenotype characteristic of a HSC. In some embodiments described herein, the HSCs is negative for (expression of) lineage markers (i.e., lin-). In some instances, the HSCs are positive for (expression of) one or more of: CD34, CD38, CD90, CD133, CD105, CD45, and c-kit. In some instances, the HSCs are CD34+ and in other instnaces, the HSCs are CD45+. In still other aspect, the HSCs are CD34+ and CD45+. In certain embodiments described herein, the phenotype of the HSCs changes once administered to the patient. Accordingly, in some embodiments, the HSCs are ones which become positive for expression of markers, e.g., circulating endothelial progenitor cell (CEP) markers (markers expressed on CEPs, e.g., CD 146, CD133, CD34, CD117, CD31).

The HSCs described herein are optionally part of a heterogeneous cell population, wherein at least 25% (e.g., at least 30%, at least 40%, at least 50%, at least 60%, at least 70%, at least 80%, at least 90%, at least 95%) of the cells in the population are CD34+ cells. The HSCs are part of a heterogeneous population of cells, wherein at least at least 70%, at least 80%, at least 90%, at least 95% of the cells are CD34+ HSCs. In some embodiments described herein, the HSCs are part of a heterogeneous population of cells, wherein at least at least 25% (e.g., at least 30%, at least 40%, at least 50%, at least 60%, at least 70%, at least 80%, at least 90%, at least 95%) of the cells are CD45+ HSCs. In some embodiments, the HSCs are part of a heterogeneous population of cells, wherein at least at least 25% (e.g., at least 30%, at least 40%, at least 50%, at least 60%, at least 70%, at least 80%, at least 90%, at least 95%) of the cells are HSCs which become CD146+ HSCs after administration to the patient.

### Further Modifying Steps of HSCs

In some aspects described herein, the HSCs are further modified after being isolated and/or purified. In one alternative, the cells are cultured *in vitro* for purposes of expanding the population of HSCs, delivering genes into the HSCs, differentiating the HSCs, or conjugating a compound, such as a therapeutic agent or a diagnostic agent, to the HSCs. Methods of carrying out these further steps are well known in the art. See, for example, Sambrook et al., Molecular Cloning: A Laboratory Manual, Cold Spring Harbor Laboratory Press, Cold Spring Harbor, NY, 2001; Ogawa et al., Blood 81: 2844-2853 (1993); U.S. Patent 7,144,731; Li et al, FASEB J 15: 586 (2001); Norol et al., Experimental Hematology 35(4): 653-661 (2007); Verhoeyen and Cosset, Gene Transfer: Delivery and Expression of DNA and RNA, eds. Friedmann and Rossi, Cold Spring Harbor Laboratory Press, Cold Spring Harbor, NY, 2007.

### Pharmaceutical compositions

The HSCs described herein are optionally formulated into a composition, such as a pharmaceutical composition. In this regard, provided herein is a pharmaceutical composition comprising the HSCs and a pharmaceutically acceptable carrier. The carrier is any of those conventionally used and is limited only by chemico-physical considerations, such as solubility and lack of reactivity with the active compound(s), and by the route of administration. The pharmaceutically acceptable carriers described herein, for example, vehicles, adjuvants, excipients, and diluents, are well-known to those skilled in the art and are readily available to the public. In one aspect the pharmaceutically acceptable carrier is one which is chemically inert to the active agent(s), e.g., the hematopoietic stem cells, and one which has no detrimental side effects or toxicity under the conditions of use. The choice of carrier will be determined in part by the particular agents comprising the pharmaceutical composition, as well as by the particular route used to administer the pharmaceutical composition. Accordingly, there are a variety of suitable formulations of the pharmaceutical composition described herein.

### Routes of Administration

In some embodiments disclosed herein, the pharmaceutical composition comprising the HSCs is formulated for parenteral administration, subcutaneous administration, intravenous administration, intramuscular administration, intraarterial administration, intrathecal administration, or interperitoneal administration. In other embodiments disclosed herein, the pharmaceutical composition is administered via nasal, spray, oral, aerosol, rectal, or vaginal administration.

Methods of administering HSCs are known in the art. See, for example, any of U.S. Patents 5423778, 5550050, 5662895, 5800828, 5800829, 5811407, 5833979, 5834001, 5834029, 5853717, 5855619, 5906827, 6008035, 6012450, 6049026, 6083523, 6206914, 6303136, 6306424, 6322804, 6352555, 6368612, 6479283, 6514522, 6534052, 6541024, 6551338, 6551618, 6569147, 6579313, 6599274, 6607501, 6630457, 6648849, 6659950, 6692738, 6699471, 6736799, 6752834, 6758828, 6787357, 6790455, 6805860, 6852534, 6863900, 6875441, 6881226, 6884427, 6884428, 6886568, 6918869, 6933281, 6933286, 6949590, 6960351, 7011828, 7031775, 7033345, 7033603, 7049348, 7070582, 7074239, 7097832, 7097833, 7135172, 7145055, 7157080, 7166280, 7176256, 7244242, 7452532, 7470425, and 7494644.

### Parenteral

In some embodiments disclosed herein, the pharmaceutical composition described herein is formulated for parenteral administration. Parenteral administration includes, but is not limited to, intravenous, intraarterial, intramuscular, intracerebral, intracerebroventricular, intracardiac, subcutaneous, intraosseous, intradermal, intrathecal, intraperitoneal, intravesical, and intracavernosal injections or infusions.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The pharmaceutical composition are in various aspects administered via a physiologically acceptable diluent in a pharmaceutical carrier, such as a sterile liquid or mixture of liquids, including water, saline, aqueous dextrose and related sugar solutions, a glycol, such as propylene glycol or polyethylene glycol, glycerol, ethers, poly(ethyleneglycol) 400, oils, fatty acids, fatty acid esters or glycerides, or acetylated fatty acid glycerides with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which are optionally used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters.

The parenteral formulations may contain preservatives or buffers. In order to minimize or eliminate irritation at the site of injection, such compositions optionally contain one or more nonionic surfactants having a hydrophile-lipophile balance (HLB) of from about 12 to about 17. The quantity of surfactant in such formulations will typically range from about 5% to about 15% by weight. Suitable surfactants include polyethylene glycol sorbitan fatty acid esters, such as sorbitan monooleate and the high molecular weight adducts of ethylene oxide with a hydrophobic base, formed by the condensation of propylene oxide with propylene glycol. The parenteral formulations are in various aspects presented in unit-dose or multi-dose sealed containers, such as ampoules and vials, and can be stored in a freeze-dried (lyophilized) condition requiring only the addition of the sterile liquid excipient, for example, water, for injections, immediately prior to use. Extemporaneous injection solutions and suspensions are in certain aspects prepared from sterile powders, granules, and tablets of the kind previously described.

Injectable formulations are disclosed herein. The requirements for effective pharmaceutical carriers for injectable compositions are well-known to those of ordinary skill in the art (see, e.g., Pharmaceutics and Pharmacy Practice, J. B. Lippincott Company, Philadelphia, PA, Banker and Chalmers, eds., pages 238-250 (1982), and ASHP Handbook on Injectable Drugs, Toissel, 4th ed., pages 622-630 (1986)).

### Cell Delivery Matrices

In some embodiments disclosed herein, the HSCs are administered via a cell delivery matrix. The cell delivery matrix may comprise any one or more of polymers and hydrogels comprising collagen, fibrin, chitosan, MATRIGEL, polyethylene glycol, dextrans including chemically crosslinkable or photocrosslinkable dextrans, and the like. The cell delivery matrix may comprise one or more of: collagen, including contracted and non- contracted collagen gels, hydrogels comprising, for example, but not limited to, fibrin, alginate, agarose, gelatin, hyaluronate, polyethylene glycol (PEG), dextrans, including dextrans that are suitable for chemical crosslinking, photocrosslinking, or both, albumin, polyacrylamide, polyglycolyic acid, polyvinyl chloride, polyvinyl alcohol, poly(n-vinyl-2-pyrollidone), poly(2- hydroxy ethyl methacrylate), hydrophilic polyurethanes, acrylic derivatives, pluronics, such as polypropylene oxide and polyethylene oxide copolymer, 35/65 Poly(epsilon-caprolactone)(PCL)/Poly(glycolic acid) (PGA), Panacryl® bioabsorbable constructs, Vicryl® polyglactin 910, and self-assembling peptides and non-resorbable materials such as fluoropolymers (e.g., Teflon® fluoropolymers), plastic, and metal.

The matrix in some instances comprises non- degradable materials, for example, but not limited to, expanded polytetrafluoroethylene (ePTFE), polytetrafluoroethylene (PTFE), polyethyleneterephthalate (PET), poly(butylenes terephthalate (PBT), polyurethane, polyethylene, polycabonate, polystyrene, silicone, and the like, or selectively degradable materials, such as poly (lactic-co-glycolic acid; PLGA), PLA, or PGA. (See also, Middleton et al., Biomaterials 21:2335 2346, 2000; Middleton et al., Medical Plastics and Biomaterials, March/ April 1998, at pages 30 37; Handbook of Biodegradable Polymers, Domb, Kost, and Domb, eds., 1997, Harwood Academic Publishers, Australia; Rogalla, Minim. Invasive Surg. Nurs. 11:6769, 1997; Klein, Facial Plast. Surg. Clin. North Amer. 9:205 18, 2001 ; Klein et al., J. Dermatol. Surg. Oncol. 1 1:337 39, 1985; Frey et al., J. Urol. 154:812 15, 1995; Peters et al., J. Biomed. Mater. Res. 43:422 27, 1998; and Kuijpers et al., J. Biomed. Mater. Res. 51:13645, 2000).

The matrix may include biocompatible scaffolds, lattices, self-assembling structures and the like, whether bioabsorbable or not, liquid, gel, or solid. Such matrices are known in the arts of therapeutic cell treatment, surgical repair, tissue engineering, and wound healing. In certain aspects, the matrix is pretreated with the HSCs. In other embodiments described herein, the matrix is populated with HSCs in close association to the matrix or its spaces. The HSCs can adhere to the matrix or can be entrapped or contained within the matrix spaces. In certain aspects, the matrix-HSCs complexes in which the cells are growing in close association with the matrix and when used therapeutically, growth, repair, and/or regeneration of the patient's own kidney cells is stimulated and supported, and proper angiogenesis is similarly stimulated or supported. The matrix-cell compositions can be introduced into a patient's body in any way known in the art, including but not limited to implantation, injection, surgical attachment, transplantation with other tissue, and the like. In some embodiments, the matrices form in vivo, or even more preferably in situ, for example in situ polymerizable gels can be used in accordance with the invention. Examples of such gels are known in the art.or the like.

The HSCs may be seeded on a three-dimensional framework or matrix, such as a scaffold, a foam or hydrogel and administered accordingly. The framework in certain aspects are configured into various shapes such as substantially flat, substantially cylindrical or tubular, or can be completely free-form as may be required or desired for the corrective structure under consideration. Two or more substantially flat frameworks in some aspects are laid atop another and secured together as necessary to generate a multilayer framework.

Examples of matrices, for example scaffolds which may be used include mats (woven, knitted, and more preferably nonwoven) porous or semiporous foams, self assembling peptides and the like. Nonwoven mats may, for example, be formed using fibers comprised of natural or synthetic polymers. In some embodiments, absorbable copolymers of glycolic and lactic acids (PGA/PLA), sold under the tradename VICRYL® (Ethicon, Inc., Somerville, N.J.) are used to form a mat. Foams, composed of, for example, poly(epsilon-caprolactone)/poly(glycolic acid) (PCL/PGA) copolymer, formed by processes such as freeze-drying, or lyophilization, as discussed in U.S. Pat. No. 6,355,699, can also serve as scaffolds. Gels also form suitable matrices, as used herein. Examples include in situ polymerizable gels, and hydrogels, for example composed of self-assembling peptides. These materials are used in some aspects as supports for growth of tissue. In situ-forming degradable networks are also suitable for use in the invention (see, e.g., Anseth, K. S. et al., 2002, J. Controlled Release 78: 199-209; Wang, D. et al., 2003, Biomaterials 24: 3969-3980; U.S. Patent Publication 2002/0022676 to He et al.). These materials are formulated in some aspects as fluids suitable for injection, then may be induced by a variety of means (e.g., change in temperature, pH, exposure to light) to form degradable hydrogel networks in situ or in vivo.

In some embodiments disclosed herein, the framework is a felt, which can be comprised of a multifilament yarn made from a bioabsorbable material, e.g., PGA, PLA, PCL copolymers or blends, or hyaluronic acid. The yarn in certain aspects is made into a felt using standard textile processing techniques consisting of crimping, cutting, carding and needling. The HSCs in certain aspects are seeded onto foam scaffolds that may be composite structures. In addition, the three-dimensional framework are molded in some aspects into a useful shape, such as a specific structure in or around the kidney to be repaired, replaced, or augmented.

The framework can be treated prior to inoculation of the HSCs in order to enhance cell attachment. For example, prior to inoculation with the HSCs, nylon matrices are treated with 0.1 molar acetic acid and incubated in polylysine, PBS, and/or collagen to coat the nylon. Polystyrene is some aspects is similarly treated using sulfuric acid.

In additional embodiments disclosed herein, the external surfaces of the three-dimensional framework is modified to improve the attachment or growth of cells and differentiation of tissue, such as by plasma coating the framework or addition of one or more proteins (e.g., collagens, elastic fibers, reticular fibers), glycoproteins, glycosaminoglycans (e.g., heparin sulfate, chondroitin-4-sulfate, chondroitin-6-sulfate, dermatan sulfate, keratin sulfate), a cellular matrix, and/or other materials such as, but not limited to, gelatin, alginates, agar, agarose, and plant gums, among others.

The scaffold may comprise materials that render it non-thrombogenic. These materials may promote and sustain endothelial growth, migration, and extracellular matrix deposition. Examples of such materials include but are not limited to natural materials such as basement membrane proteins such as laminin and Type IV collagen, synthetic materials such as ePTFE, and segmented polyurethaneurea silicones, such as PURSPAN® (The Polymer Technology Group, Inc., Berkeley, Calif.). These materials can be further treated to render the scaffold non-thrombogenic. Such treatments include anti-thrombotic agents such as heparin, and treatments which alter the surface charge of the material such as plasma coating.

The pharmaceutical composition comprising the HSCs may comprise any of the components of a cell delivery matrix, including any of the components described herein.

### Dose

For purposes herein, the amount or dose of the pharmaceutical composition administered are sufficient to effect, e.g., a therapeutic or prophylactic response, in the subject or animal over a reasonable time frame. For example, the dose of the pharmaceutical composition is sufficient to treat or prevent renal ischemia reperfusion injury in a period of from about 1 to 4 days or longer, e.g., 5 days, 6 days, 1 week, 10 days, 2 weeks, 16 to 20 days, or more, from the time of administration. In certain embodiments, the time period is even longer. The dose is determined by the efficacy of the particular pharmaceutical composition and the condition of the animal (e.g., human), as well as the body weight of the animal (e.g., human) to be treated.

Many assays for determining an administered dose are known in the art. In some embodiments, an assay which comprises comparing the extent to which HSCs are localized to an injured kidney upon administration of a given dose of such HSCs to a mammal among a set of mammals of which is each given a different dose of the HSCs is used to determine a starting dose to be administered to a mammal. The extent to which HSCs are localized to an injured kidney upon administration of a certain dose can be assayed by methods known in the art, including, for instance, the methods described herein.

Additionally or alternatively, an assay which comprises comparing the extent to which a particular dose of HSCs cause attenuation of kidney peritubular capillary loss, regeneration of tubular epithelial cells, prevention of long-term fibrosis, reduction of mortality, or improvement of kidney function after a kidney injury can be used to determine a starting dose to be administered to a mammal. Such assays are described herein under EXAMPLES.

The dose of the pharmaceutical composition also will be determined by the existence, nature and extent of any adverse side effects that might accompany the administration of a particular pharmaceutical composition. Typically, the attending physician will decide the dosage of the pharmaceutical composition with which to treat each individual patient, taking into consideration a variety of factors, such as age, body weight, general health, diet, sex, therapeutic agent(s) of the pharmaceutical composition to be administered, route of administration, and the severity of the condition being treated. By way of example and not intending to limit the invention, the dose of the pharmaceutical composition can be such that at least about 0.5 x 10⁶ (e.g., at least about 1 x 10⁶, 1.5 x 10⁶, 2 x 10⁶, 2.5 x 10⁶, 3.0 x 10⁶, 5.0 x 10⁶, 10⁷, 10⁸) HSCs are administered to the patient.

### Timing of Administration

In methods described herein, the HSCs are administered to the patient at a time in reference to the time of injury to the kidney. Administration of the HSCs is delayed; that is, the HSCs are not administered immediately after the kidney injury and are administered at least 20 hours post injury.

In some aspects described herein, the HSCs are administered to the patient at the beginning of the repair phase of the kidney injury. The term "repair phase of the kidney injury" as used herein refers to the time after injury at which a renal regenerative response is observed, as represented by, e.g., repopulation of the existing nephron after cells have been destroyed, lining of the tubules with basophilic, flattened squamous cells, restoration of normal morphology of tubule cells, epithelial cell dedifferentiation, movement, proliferation, or redifferentiation, restoration of functional integrity of nephron, restoration of renal function. The repair phase of the kidney is well documented in mammals. See, for example, Reimschuessel, ILAR J 42: 285-291 (2001). The HSCs may be administered at least 20 hours, at least about 21 hours, at least about 22 hours, at least about 23 hours, at least about 24 hours, at least about 25 hours, at least about 26 hours, at least about 28 hours, at least about 30 hours, at least about 32 hours, at least about 32 hours, at least about 34 hours, at least about 36 hours, at least about 38 hours, at least about 40 hours, at least about 42 hours, at least about 44 hours, at least about 46 hours, at least about 48 hours, at least about 50 hours, at least about 52 hours, at least about 54 hours, at least about 56 hours, at least about 58 hours, at least about 60 hours, at least about 62 hours, at least about 64 hours, at least about 66 hours, at least about 68 hours, at least about 70 hours, at least about 72 hours) post-injury.

The HSCs may be administered to the patient at a timepoint as described above and before 7 days, before about 6 days, before about 5 days, before about 4 days, before about 3 days) post injury. In some embodiments described herein, the HSCs are administered to the patient at about 24 hours post-injury, or some time thereafter, but before 7 days post-injury.

The HSCs may be administered after X post-injury and before Y post-injury, wherein X is selected from a group consisting of about 20 h, 21 h, 22 h, 23 h, 24 h, 25 h, 26 h, 27 h, 28 h, 29 h, 30 h, 31 h, 32 h, 33 h, 34 h 35 h, 36 h, 40 h, 48 h, 52 h, 58 h, 64 h, 72 h, 3.5 d, 4 d, 5 d, 6 d, wherein Y is 1 week, and wherein X is less than Y. In some aspects described herein, the HSCs are administered about 20, 21, 22, 23, 24 hours post-injury.

In some embodiments described herein, the HSCs are administered to the patient more than once. The HSCs may be administered once daily, twice daily, 3X, 4X daily, once weekly, once every 2, 3, 4, 5, 6, 8, 9, 10, 11, 12, 13, or 14 days, or once monthly. In some embodiments described herein, the HSCs are administered after about 24 hours (e.g., at 24 hours) post-injury and administered again after about 48 hours (e.g., at 48 hours) post-injury.

### Controlled Release Formulations

The pharmaceutical composition may be modified into a depot form, such that the manner in which the pharmaceutical composition is released into the body to which it is administered is controlled with respect to time and location within the body (see, for example, U.S. Patent No. 4,450,150). Depot forms are in various aspects, an implantable composition comprising the therapeutic or active agent(s) and a porous or non-porous material, such as a polymer, wherein the HSCs is encapsulated by or diffused throughout the material and/or degradation of the non-porous material. The depot is then implanted into the desired location within the body and the HSCs are released from the implant at a predetermined rate.

Accordingly, the pharmaceutical composition may be modified to have any type of in vivo release profile. In some aspects, the pharmaceutical composition is an immediate release, controlled release, sustained release, extended release, delayed release, or bi-phasic release formulation.

### Conjugates

In some embodiments described herein, the HSCs are attached or linked to a second moiety, such as, for example, a therapeutic agent or a diagnostic agent. The HSCs of these embodiments act as a targeting agent, since the HSCs are able to specifically localize to injured kidney tissue. Accordingly, a composition comprising HSCs attached to a therapeutic agent of a diagnostic agent is described. Suitable therapeutic agents and diagnostic agents for purposes herein are known in the art and include, but are not limited to, any of those mentioned herein.

### Combinations

The pharmaceutical compositions described herein, including the conjugates, are administered by itself in some embodiments. In other embodiments described herein, the pharmaceutical compositions, including the conjugates, are administered in combination with other therapeutic or diagnostic agents. In some embodiments described herein, the pharmaceutical composition is administered with another therapeutic agent known to treat a renal disease or renal medical condition, including, for example, a cytokine or growth factor, an anti-inflammatory agent, a TLR2 inhibitor, a ATF3 gene or gene product, and a mineralocorticoid receptor blocker (e.g., spironolactone), a lysophosphatidic acid, 2-methylaminochroman (e.g., U83836E), a 21-aminosteroid (e.g., lazoroid (U74389F)), trimetazidine, angiotensin converting enzyme (ACE) inhibitors or angiotensin receptor blockers (ARB), and suramin.

In some embodiments described herein, the HSCs are administered with other additional therapeutic agents, including, but not limited to, antithrombogenic agents, anti-apoptotic agents, anti-inflammatory agents, immunosuppressants (e.g., cyclosporine, rapamycin), antioxidants, or other agents ordinarily used in the art to treat kidney damage or disease such as eprodisate and triptolide, an HMG-CoA reductase inhibitor (e.g., simvastatin, pravastatin, lovastatin, fluvastatin, cerivastatin, and atorvastatin), cell lysates, soluble cell fractions, membrane-enriched cell fractions, cell culture media (e.g., conditioned media), or extracellular matrix trophic factors (e.g., hepatocyte growth factor (HGF), bone morphogenic protein-7 (BMP-7), transforming growth factor beta (TGF-β), matrix metalloproteinase-2 (MMP-2), and basic fibroblast growth factor (bFGF).

### Patient types

With regard to the methods described herein, the patient is any host. In some embodiments described herein, the host is a mammal. As used herein, the term "mammal" refers to any vertebrate animal of the mammalia class, including, but not limited to, any of the monotreme, marsupial, and placental taxas. In some embodiments described herein, the mammal is one of the mammals of the order Rodentia, such as mice and hamsters, and mammals of the order Logomorpha, such as rabbits. In certain embodiments, the mammals are from the order Carnivora, including Felines (cats) and Canines (dogs). In certain embodiments described herein, the mammals are from the order Artiodactyla, including Bovines (cows) and S wines (pigs) or of the order Perssodactyla, including Equines (horses). In some instances, the mammals are of the order Primates, Ceboids, or Simoids (monkeys) or of the order Anthropoids (humans and apes). In particular embodiments, the mammal is a human.

The following examples are given merely to illustrate the present invention and not in any way to limit its scope.

### EXAMPLES

### EXAMPLE 1

The following materials and methods were used in Examples 2-7.

### Animals

Male immune deficient non -obese diabetic (NOD/SCID) mice (NOD.CB17-*Prkdc^{scid}*/J) (Jackson Laboratories, Bar Harbor, ME) were used at the age of 8-10 weeks. Note these mice are not diabetic, but lack functional T and B cells. All mice were maintained in filter top cages and received sterilized food and acidified water. All experimental protocols were approved by the Harvard Center for Animal Research and Comparative Medicine.

### Human Peripheral Blood CD34+ Cell Purification And Tracking

Human peripheral blood CD34+ stem cells were selected from granulocyte colony stimulating factor (G-CSF) mobilized apheresis products obtained from normal healthy adult donors (catalog# mPB026, AllCells, Berkeley, CA). Briefly, G-CSF (10µg/kg/day) was administered to donors for 5 consecutive days to mobilize CD34+ cells from the bone marrow into peripheral circulation. Donors underwent apheresis on days 5 and 6 to collect mobilized peripheral blood mononuclear cells. CD34+ cells were highly enriched from the apheresis product using ISOLEX 300i Magnetic Cell Positive Selection System (version 2.5, Baxter Healthcare, Deerfield, IL, USA) according to the protocol provided with the instrument's User Manual. Purified cells were characterized by flow cytometry (see below). Enriched, selected cells were maintained in RPMI with 0.5% human serum albumin at 25 °C and used within 48h. To test viability, aliquots of 2 x10⁵ cells were labeled with 7-AAD (20 µg/ml, 20min, 4 °C in 100 µl PBS), washed with FACS buffer (PBS/5%BSA), then analyzed by Flow cytometry. In some experiments, in order to track HSCs following systemic administration, human CD34+ cells were labeled with the green fluorescent tracer 5-chloromethylfluorescein diacetate (CMFDA, Invitrogen) using 1µg/10⁷ cells for 30 minutes at 25 °C in 10 ml of RPMI. Excess CMFDA was quenched after centrifugation (250xg 5min) by resuspending in 10ml 5%BSA (ultrapure) (Sigma) in RPMI (Invitrogen). After further centrifugation cells were resuspended in 1%BSA/RPMI (12.5 x 10⁶/ml). CMFDA labeling did not yield any changes in viability detected using 7-AAD (not shown).

### Animal Model

Ischemia-reperfusion injury of the kidney was modified from methods previously described [1]. In brief, on day 0, kidneys of anesthetized ma le mice (8-10 weeks) were exposed through surgical incisions to the flanks, and at core temperature of 36.8-37.3 °C a surgical clamp was placed across the renal artery and vein of either one or both kidneys. The kidneys were confirmed to become dusky, then replaced in the retroperitoneum for 27 minutes (unilateral model) or 25 minutes (bilateral model). Clamps were removed and reperfusion to kidneys was confirmed visually, and wounds closed. To test the effect of human HSCs, these mice with unilateral IRI kidney injury were divided into two groups. In the treatment group (n = 6-10/group), on days 1 and 2 after kidney injury, 200 µl of cell suspension containing 2.5 x 10⁶ human CD34+ cells labeled with CMFDA was infused intravenously through the tail vein. In the control group, mice were only given vehicle. Mice were sacrificed on days 3, 5, 7, 14 and 28 IRI of the kidney.

### Renal Function

To evaluate renal function, mice with bilateral IRI kidney injury (d0) were randomly divided into two groups. The treatment group (n = 10), received 2.5 x 10⁶ human HSCs by intravenously tail vein infusion on days 1 and 2. The control group (n = 16) received vehicle only. Plasma creatinine was analyzed from blood samples were taken from the tail vein on days 1, 2, 5, 7, 14 and 28 after injury using Methods previously described [1].

### Tissue Preparation, Immunostaining, Imaging And Quantification Of Injury And Repair

Mice were perfused with ice cold PBS then organs fixed in periodate-lysine paraformaldehyde (PLP) solution for 2h followed by 18% sucrose 16h, then preserved in optimal cutting temperature (OCT) medium (an embedding medium used for frozen tissue to ensure Optimal Cutting Temperature and to embed tissue before sectioning on a cryostat) (-80 °C) [2], or tissue for light microscopy was fixed in 10% neutral-buffered formalin for 12h, transferred to 70% ethanol, then processed for paraffin sections (3mm) and sections and stained with periodic acid-Schiff (PAS) or picrosirius red stain 2. Immunofluorescence labeling was performed on 5mm cryosections. To detect infused human cells in kidneys, spleen and heart, either antibodies against human leukocyte antigens with no cross-reactivity to mouse antigens were used or fluorescence of CMFDA was used (up to d7). The following antibodies were used employing methods described elsewhere [1,2]: anti -human leukocyte antigen class I (HLA)-ABC (FITC, 1:200, eBioscience), anti-human CD45 (FITC, 1:200, eBioscience), rat-anti-human CD45 (1:200, Abcam), anti-human CD68 (FITC, 1:200, eBioscience), anti-human CD3 (FITC, 1:200, eBioscience), anti-human CD31 (FITC, 1:200, eBioscience), rabbit anti-human vWF (1:200, Abcam), anti-human CD 146 (FITC, 1:100, Abcam), biotin-anti-human CD 13 3 (1:100, Miltenyi), rabbit-anti-human-CD 13 3 (1:100, CellSignaling), goat-anti-human KDR (1:100, R&D Systems), and rabbit-anti-human KDR (1:100, NeoMarkers), followed by rabbit-anti FITC (1:200, Invitrogen), anti-rat Cy3 or anti-rabbit Cy3 or anti-goat Cy3 (1:400, Jackson Immunosresearch) or anti-avidin Cy3 (1:3000, Jackson Immunosresearch). To label mouse vasculature rat-anti-mouse CD31 (1:200, eBioscience), which does not cross-react with human antigen was applied, followed by anti-rat Cy3 (1:400, Jackson Immunosresearch). Sections were post- fixed with 1% paraformaldehyde (PFA), then mounted in Vectashield with DAPI. Peritubular capillary loss and tubule injury were determined by assessing anti-CD31-Cy3 labeled kidney sections or PAS stained paraffin sections respectively using a blinded scoring method as reported previously [3]. In brief, images were captured by digital imaging (X200) sequentially over the entire sagittal section incorporating cortex and outer medulla (10-20 images). Each image was divided into 252 squares by a grid. To calculate peritubular capillary loss, each square without a peritubular capillary resulted in a positive score; the final score presented as a percentage positive score. To assess the tubular injury, each square the presence of tubule injury (tubule flattening, necrosis, apoptosis or presence of casts) resulted in a positive score. The final score is the percentage of squares with positive score per image, which was averaged for all images from the individual kidney. Epifluorescent images were taken with a Nikon TE2000 microscope, CoolSnap camera (Roper Scientific, Germany) and processed using IP lab software (BD Biosciences, San Jose, CA). Confocal images were generated using a Nikon C1 D-Eclipse confocal microscope. Projection images were generated from 10 Z- stack images that were acquired at 0.1 mm steps. To allow comparison between sections, all confocal settings including were kept constant between sections.

### Flow Cytometric Analysis And Sorting

Isolex-enriched CD34 cells were analyzed using the following human antibody combinations: anti-CD31-FITC (1:100, BD), anti-CD146-PE (1:100, BD), anti-KDR- FITC (1:100, R&D Systems), anti-CD45-FITC (1:100, BD), anti-CD140b-Alexa Fluor 488 (1:100, BD), anti-CD29-PE (1:100, BD), anti-CD105-FITC (1:100, R&D Systems), anti-CD34-PE (1:100, BD), anti-CD99-FITC (1:100, BD), anti-CD144-PE (1:100, R&D Systems), anti-CD38-FITC (1:100, BD), anti-CD14-FITC (1:100, BD), anti-CD64-PE (1:100, BD), anti-CD61-PerCP (1:100, BD) anti-CD133-APC (1:100, Miltenyi), antiCXCR4-APC (1:100, BD), anti-CD90-APC (1:100, BD), anti-CD 117-APC (1:100, BD), anti-VEGFR1-APC (1:100, R&D Systems), using methods previously described [1]. Full characterization of HSCs will be documented elsewhere (D.M. & A.C. unpublished). Single cells were prepared from kidney, spleen and bone marrow as previously described [2]. In brief, single cells (1 x 10⁵) from kidney, spleen and bone marrow were resuspended in FACS buffer and incubated with antibodies against human CD45 (FITC, 1:200, eBioscience) and mouse CD 11b (PE, 1:200, eBioscience) for 30 minutes. After washing with FACS wash buffer, and resuspending in 200 µl FACS buffer, cells were analyzed using BD FACSCalibur flow cytometer. The human HSCs labeled with CMFDA on day 2 after injection were sorted directly by FACS sorting using FACSaria [2]. Sorted CMFDA+ cells from kidney were immediately lysed and RNA purified using RNA Easy (Qiagen) system, for real time PCR.

### Real Time PCR

Total RNA was generated from tissue and cells using a kit (RNA Easy Qiagen), according to the manufacturer's instructions. Purity determined by A260 to A280. cDNA was synthesized from 1 µg of total RNA using iScript and primers comprising random hexamers and poly dT [2]. Real-time PCR of human and mouse samples was performed using an ABI7900HT sequence detection system (PerkinElmer Life Sciences, Boston, Applied BioSystems, Foster City, CA) in the presence of SYBR -Green (SYBR Green PCR kit; Qiagen) using methods previously described [4]. Primer/probe sets specific for human GAPDH, HPRT1, Angiopoietin 1 (ANGPT1), Fibroblast growth factor 2 (FGF2), Hepatocyte Growth Factor (HGF), Insulin-like growth factor 1(IGF1), interleukin-8 (IL8), Platelet-derived growth factor b (PDGFb), transforming growth factor b 1 (TGFb 1), Vascular endothelial growth factor (VEGF), TIE1, were from Sabiosciences. Equal amounts of cDNA were used for RT-PCR reaction and mixed with ready to use reaction mix (Sabiosciences). All of the reactions were performed in triplicate. Optimization of the real-time PCR was performed according to the manufacturer's instructions. For standard curve determination, a pool of all the samples, serially diluted in four log2 steps and run in parallel to the samples, were used. The total volume of each reaction was 20 µl, containing 300 nM forward and 300 nM reverse primer and 125ng of cDNA. Appropriate negative controls were run for each reaction.

### Statistical Analysis

All values are given as mean ± standard deviation (SD). Mantel-Cox Log-rank test was used to analyze survival. Comparisons between two groups were carried by unpaired t-test (two tailed). Paired t test was used to compare directly the left (IRI) and right (control) kidney of an animal or diseased mouse compared with sham operated mouse. P values less than 0.05 were considered significant in all statistical tests.

### EXAMPLE 2

### Characterization Of Isolex-Purified G-Csf-Mobilized Hematopoietic Stem Cells

CD34+ enriched leukocytes from hematopoietic stem cell-mobilized human donors were analyzed for viability and purity. More than 99% of HSCs were viable by 7-AAD exclusion (not shown). More than 96% of leukocytes were CD45+, CD34+ indicating they were hematopoietic stem cells (HSCs) (see Table 1). A minority expressed CD34 but not CD45. Further characterization of the enriched leukocytes was performed using the cell surface markers CD14, CD34, CD146, CD133, CD31, VEGFR2 for confirmation of multi-lineage potential and identification of putative endothelial progenitors (see Table 2) [19]. The characterization indicates that in addition to HSCs, mobilized human peripheral blood CD34+ cells contain small numbers of circulating endothelial progenitor cells (CEPs) and rare circulating endothelial cells (CECs).

**TABLE 1**

| **Human CD34+ enriched mobilized peripheral blood stem cells (n = 6/group).** | | | |
|---|---|---|---|
| **Markers** | **CD34+** | **CD34+CD45-** | **CD34-CD45+** |
| **Average** | 96.44 | 0.08 | 2.96 |
| **SD** | 0.84 | 0.13 | 0.89 |

**TABLE 2**

| **CEC and CEP phenotypes in human CD34+ enriched mobilized peripheral blood stem cells (n = 6/group)** | | | | | |
|---|---|---|---|---|---|
| | **CEPs** | | | | **CECs** |
| **Markers** | **CD34+ CD14+** | **CD34+KDR+ CD133+** | **CD34+KDR+ CD146-CD31-** | **CD34+CD133+ CD45+CD146+** | **CD34+CD133-CD146+CD31+** |
| **Average** | 0.05 | 0.30 | 0.05 | 0.07 | 0.05 |
| **SD** | 0.04 | 0.22 | 0.07 | 0.11 | 0.06 |

### EXAMPLE 3

### Human Hematopoietic Stem Cells Are Recruited To Kidney During Repair Following Ischemia Reperfusion Injury

To study the effect of human HSCs on kidney repair we initially determined whether they could be recruited to the injury kidney. In preliminary studies I.V. infusion of 2.5 x 10⁶ HSCs labeled with CMFDA prior to injury did not result in significant recruitment 24h after injection (data not shown). Next we infused CMFDA-labeled HSCs on day 1 and 2 after kidney IRI, and looked in the kidney 3, 5, 7 days following injury (Figure 1A, B) where many recruited HSCs could be detected in the kidney parenchyma. Many were localized within peritubular capillaries (PTC), but some were detected outside of the confines of the capillaries in a perivascular location (Figure 1C). We also noticed that following unilateral IRI there was a small but significant recruitment of HSCs to the uninjured kidney (Figure 1B). However we could not detect any HSCs in the heart (not shown) indicating that this was specific recruitment of HSCs to the uninjured and injured kidney. Due to concern that CMFDA might become diluted with time we infused unlabeled HSCs into mice on d1 and d2 following injury. These unlabeled cells were detected by antibodies against HLA class I (Figure 1C, D). HLA-I positive cells were readily detected in the kidneys at all time points but notably there was persistence of HLA-I+ cells in the kidney 14 and 28 after IRI (Figure 1D). As expected, HSCs were also identified in spleen and bone marrow (Figure 1E-H), and there was persistence of HSCs in the marrow, with evidence on d7 following IRI that HSCs in the bone marrow had induced the myeloid marker CD11b (Figure 1G) suggesting that HSCs had engrafted the mouse bone marrow and that the mice were now chimeric.

### EXAMPLE 4

### Systemic Human Hematopoietic Stem Cell Therapy Reduces Mortality And Improves Kidney Function Following Ischemia Reperfusion Injury

To determine whether HSC recruitment to the injured kidney had any functional consequence during repair, we subjected mice to bilateral IRI (day 0), followed by intravenous infusion of human HSCs on d1 and d2. Plasma creatinine was assessed in sham surgery mice (d0, plasma creatinine value is 0.05 ± 0.06) and on d1, d2, and d7 following IRI. Bilateral kidney IRI resulted in significant increase in serum creatinine at 24 hours and peaked at 48h (Figure 2A). Although plasma creatinine levels at 24 hours (time of first injection) were no different in treatment and vehicle groups, there was a marked and significant decrease in plasma creatinine at 48h in mice that had received HSCs (Figure 2A), while the vehicle group of mice had persistently highly elevated plasma creatinine levels at this time. By d7, in mice that had survived, both vehicle and treatment groups showed similar levels of plasma creatinine. This is not surprising since the IRI model is a recovery model. In the vehicle group, however, only 50% of mice survived to day 7 (Figure 2B), whereas 90% of mice that received human HSCs survived to day 7 (Figure 2B). The surviving numbers in the two groups can be seen in Figure 2B. These striking findings indicate that human HSCs both promote kidney repair/regeneration and enhance survival.

### EXAMPLE 5

### Human Hematopoietic Stem Cell Therapy Attenuates Kidney Peritubular Capillary Loss, Promotes Tubular Epithelial Regeneration And Prevents Long-Term Fibrosis Following Ischemia Reperfusion Injury

To study the mechanism by which HSCs promote kidney repair we analyzed kidney sections for loss of peritubular capillaries (PTCs) and persistence of tubule injury (Figure 3). Analysis of mCD31-labeled PTCs by morphometry revealed that HSC treatment prevented PTC loss (Figure 3A-C) during repair. However notably the PTC loss after 14 and 28 days was not different indicating that there are endogenous factors that promote regeneration of PTCs, but that HSC therapy attenuates early loss of vasculature. Similarly, HSC therapy attenuated persistence of tubule injury during the repair phase of this model of IRI (Figure 3D-F), suggesting that HSCs are either directly or indirectly promoting tubule regeneration. We have previously demonstrated that kidney IRI can lead to persistent interstitial fibrosis, a harbinger of chronic kidney disease and strongly associated with progressive long-term loss of kidney function [14, 20-22]. To test whether systemic infusion of HSCs during repair of the injured kidney affected long-term consequences of injury we quantified interstitial fibrosis (Figure 3G-I). In vehicle treated mice, interstitial fibrosis progressively accumulated in the four weeks following injury but in those mice that had received HSCs interstitial fibrosis was attenuated by d28.

### EXAMPLE 6

### Human Hematopoietic Stem Cells Acquire Early Lymphoid And Myeloid Differentiation And Endothelial Progenitor Cell Markers In The Kidney Following Ischemia Reperfusion Injury

HSCs are the source of myeloid, erythroid, megakaryocyte and lymphoid lineage cells. We noted that while many HSCs were recruited to kidneys on d3 after injury the number of retained cells fell progressively through d7, but thereafter increased again up to d28 after injury (Figure 1). We labeled kidneys for human lymphoid and myeloid commitment markers (Figure 4). As early as d3 after injury many of the recruited HSCs had acquired CD68 or CD3 and this induction was similar in both uninjured and injured kidney (Figure 4). While none of the HSCs recruited to kidney through d7 acquired monocytic nuclear morphology, these data suggest that there is early local commitment toward myeloid and lymphoid lineages within the kidney. The number of human cells in the kidney increases late after injury. We observed occasional human cells with characteristic nuclei of neutrophils in d14 and d28 post IRI kidneys (not shown). These findings together with findings of BM chimerism suggest that the late increase in human cells in the kidney either reflects bone marrow chimerism or reflects local differentiation of mature cell types in the kidney.

To investigate further the local differentiation of HSCs in the kidney, sections labeled with markers VEGFR2 (KDR), CD146 and CD133, and cell surface expression was compared with stem cells prior to infusion (Table 3). While few mobilized enriched HSCs expressed KDR or CD146, the majority expressed CD133. However in the kidney d3 post IRI there was a phenotypic switch since nearly all recruited HSCs expressed CD146, but none expressed CD133. The expression of KDR was similar in mobilized, enriched HSCs compared with those recruited to kidney. Since CD 146 has been associated with CEP functions, our findings suggest that the kidney promotes HSC differentiation toward CEP type function.

**TABLE 3**

| **Percentage of HSCs expressing cell surface markers before IV injection and following recruitment to kidney at d3 and d5 post IRI (n = 5/group)** | | | |
|---|---|---|---|
| Markers | CD34+KDR+ | CD34+CD146+ | CD34+CD133+ |
| hCD34+before IV | 0.32±0.15 | 0.12±0.16 | 73.9±5.97 |
| D3 after IRI | 1.0±1.7 | 97.9±3.63 | 0 |
| D5 after IRI | 0 | 47.6±4.1 | 0 |

### EXAMPLE 7

### Human Hematopoietic Stem Cells Contribute To Vascular Repair Primarily By Paracrine Mechanisms

To study further the role of HSCs to support neovascularization, we initially determined whether HSCs had differentiated into endothelial cells. Using the human-specific antibodies against CD31 and human vWF, two markers of endothelial cells, we identified human CD31+ cells in injured kidneys at day 7, 14 and 28, but not at earlier timepoints (Figure 5A, B, C). Therefore CD31 expression did not coincide with maximal repair. Occasional CD31+ HSCs lacked CD45 expression and were found in the PTC wall with morphology consistent with endothelial cells (Figure 5A). However the vast majority of CD31+ human cells also co-expressed CD45 (Figure 5B) or were located in the interstitium with leukocyte morphology, consistent with CD31 expression by lymphocytes and monocytes, and indicating that human CD31 is not a specific marker of endothelium. Parallel studies using anti human-vWF antibodies (that did not cross react with mouse vWF) also identified very rare vWF+ human cells which lacked CD45 expression (Figure 5D), adding weight to the observation that occasional human cells do become functioning endothelial cells. Since these investigations provided evidence for only a minor contribution of human CD34+ cells to direct capillary regeneration, but there was marked induction of the CEP marker CD 146 in all HSCs (Table 1), we tested whether HSCs were functioning by paracrine mechanisms. This was particularly tractable given the intra and perivascular locale of HSCs in the kidney following injury. To study this further we purified CMFDA-labeled HSCs that had been recruited to the kidney on d4 post IRI and analyzed their human specific transcriptional profile by RT-PCR comparing it to the transcriptional profile of homogeneic HSCs prior to systemic injection into mice. HSCs generated high levels of transcripts for pro-angiogenic cytokines including ANG-1, FGF-2, and VEGF-A, and in addition generated high levels of HGF recognized for its role in epithelial regeneration (Figure 6). Strikingly, those HSCs that were recruited to the kidney exhibited highly similar transcriptional activity for the pro-angiogenic cytokines, further supporting a paracrine role in angiogenesis.

### EXAMPLE 8

### Discussion of the Data Presented Herein

Acute kidney injury in humans continues to confer high mortality and has limited therapeutic options, therefore identifying potential regenerative approaches, as new therapeutic strategies are highly desirable. In addition emerging evidence indicates that acute kidney injury in humans is a harbinger of chronic kidney disease characterized by inflammation, vasculopathy, epithelial atrophy, fibrosis and progressive loss of function leading to organ failure [2, 14, 22, 23]. New strategies that attenuate kidney injury or enhance repair and regeneration will not only have short-term impact but conceivably will alter the long term course for kidney function. The long-term consequences for such therapies will impact not only kidney disease but also cardiovascular diseases since chronic kidney disease is an independent risk factor for cardiovascular diseases [4]. Recently, adult human peripheral blood CD34+ cells as well as HSCs have been reported to promote vasculogenesis and osteogenesis following stroke and bone injury [16, 24]. Furthermore, CD34+ cells are capable of expansion and mobilization into the peripheral circulation in the presence of exogenously applied G-CSF [25-27], making HSCs readily available, and strengthening the rationale of clinical cellular therapy.

In the present study, we demonstrated that human HSCs administered systemically 24h following kidney injury were selectively recruited to injured kidneys and localized prominently in and around vasculature. This recruitment was associated with enhanced repair of the microvasculature, tubule epithelial cells, enhanced functional recovery and increased survival and additionally, prevented long-term fibrosis. HSCs induced early lymphoid and myeloid commitment markers in the kidney, acquired CEP markers but retained synthesis of high levels of pro-angiogenic transcripts following recruitment to the kidney. Although the purified HSCs contained small numbers of circulating endothelial progenitors and occasional circulating endothelial cells prior to recruitment to the kidney and kidney-recruited HSCs induced CD 146 consistent with CEP differentiation, we identified very few human endothelial cells in the mouse capillary walls. Taken together these data indicate that HSC-mediated renal repair is by paracrine mechanisms rather than replacement of vasculature (Figure 7).

Human HSCs were selectively recruited into injured kidney in the model of unilateral kidney IRI, indicating that injured kidney can selectively recruit HSCs that are in the peripheral circulation. Selective recruitment of human HSCs to post IRI kidney indicates local release of chemokines, including stromal derived factor-1 (SDF-1) and its receptor CXCR4, may be important and the transcription factor hypoxia inducible factor-1 (HIF-1) may play a role in regulating local chemokine induction [28, 29]. It was notable that systemic administration of HSCs at the onset of injury (d0) led to poor recruitment of HSCs, but that delayed administration of HSC at the beginning of the repair phase was highly effective in triggering recruitment. This recruitment pattern is similar to monocyte influx to the kidney, and unlike neutrophil recruitment, which suggests that additional monokines may play a role in HSC recruitment. Our prior studies in mice provided no evidence for endogenous HSC mobilization from the bone marrow or recruitment to the kidney, simply in response to IRI, indicating that there is an inadequate endogenous signal for recruitment of HSCs from their normal niche in the bone marrow [30]. Since injection of HSCs into the peripheral circulation results in effective recruitment to the kidney, HSC therapy overcomes a normal block in release from the bone marrow niche. Small numbers of human HSCs were also recruited to the uninjured kidney in the unilateral model of IRI. No HSCs were recruited to heart or gut in the same mice, or to kidneys of healthy mice (not shown). In response to unilateral IRI, the uninjured kidney undergoes compensatory changes which included hypertrophy and hyperplasia. It is possible therefore that HSC recruitment to the uninjured kidney either promotes angiogenesis or plays a protective role in the absence of injury.

HSCs were detected in the kidney through d14 and d28 after IRI, using antibodies against HLA-class-I antigens. There was a bimodal distribution of HSC retention in the kidney with time, with the nadir occurring at about seven days. We noted that the mice developed bone marrow chimerism, and that at d14 and d28 (but not earlier) some of the human cells in the kidney were neutrophils. It is likely therefore that for the first 7d-10d during repair of the kidney the HSCs remained as stem cells, early committed cells or CEPs, and slowly disappeared from the kidney as repair progressed, to be subsequently replaced with mature leukocytes which were recruited from bone marrow rather than deriving from the original systemic circulation HSCs. The late increase in human leukocytes in the kidney together with late expression of CD31 and appearance of human neutrophils are consistent with leukocyte recruitment from the chimeric bone marrow. However, our data strikingly point to HSC infusion on d1 and d2 of disease resulting in long-term impact on fibrosis. It is unclear from these current studies whether a reduction in long-term fibrosis reflects improved early vascular repair or whether it reflects a persistent population of reparative human HSCs in the kidney at late time-points.

Ischemic injury in the kidneys is characterized by epithelial injury. Less well described is the loss of peritubular capilliaries (PTCs). But, data derived from several severe acute kidney injury models (ischemia, toxin, transient angiotensin II) demonstrate capillary loss that typically precedes the development of prominent fibrosis [14, 15, 31], and neoangiogenesis may be a central process in preservation of vascular structure and restoration of organ function [12,13, 32,33]. We also show in these studies that following IRI there is marked loss of PTCs with only relatively mild renal injury and that although there is significant regeneration of these PTCs during repair, there is persistent loss of vasculature one month after injury, indicating that the kidney has an inherent defect in revascularization after injury [14], unlike other organs such as skin. Our studies show unequivocally that HSCs attenuate that loss of PTCs in the kidney during the repair, and this is associated with both rapid functional recovery of the kidney and enhanced survival of mice. In our unilateral IRI model, HSC-mediated regeneration of PTCs did not attenuate the long-term persistent PTC loss at 28 weeks, but nevertheless impacted on recovery and survival seen in the bilateral IRI model, pointing to early vascular repair as a central process in renal repair. Despite the efficacy HSC-mediated vascular repair being restricted to early timepoints after injury, there is nevertheless prevention of fibrosis progression in the kidney at one month after injury. Further studies will be required to understand whether this long-term effect of early HSC infusion is due to enhanced pericyte-endothelial cell interactions which may be a central interaction in the development of interstitial fibrosis [34]. In preliminary studies late administration of HSCs to mice 14 days post IRI kidney resulted in poor recruitment and little evidence of enhanced vascular repair (not shown), indicating that there is a restricted period post injury during which HSCs can be efficacious.

Although the kidney IRI model is characterized by severe injury and repair of the tubule epithelial cells, particularly the S3 segment of the proximal tubule cells, it is likely that without PTC regeneration those injured tubules will not regenerate successfully due to persistent ischemia [14, 35]. Our studies also showed that HSCs promoted epithelial regeneration, as assessed by tubule injury score and functional recovery. HSCs generated high levels of transcripts for pro-angiogenic factors, and their locale in the kidney (intravascular and perivascular) suggests a primary role in vascular repair, which secondarily promotes epithelial repair. However high level transcripts for HGF which is known to have pro-reparative effects directly on epithelia and also the epithelial reparative cytokine WNT7b (not shown) (Lin et al. manuscript in submission) suggests that HSCs might have direct paracrine role on epithelial repair, independently of PTC repair.

It is also interesting that HSCs rapidly induced CD3+ and CD68+ expression in the repairing kidney. Increasing evidence points to reparative roles for both T cells and monocyte derived cells in the kidney following injury [36, 37]. Therefore it is also possible that HSCs are locally differentiating into reparative T cells and reparative macrophages. Further studies will be required with determine the differences between kidney recruited HSCs and mature T cells and monocytes from the peripheral blood [36, 37].

The role of circulating endothelial cells (CECs) and CEPs in endothelial regeneration by directly forming mature endothelial cells has been the subject of considerable study [18, 38]. In fact we have previously reported in mouse bone marrow chimeras that a minority of endothelial cells derive from the chimeric bone marrow following kidney IRI and repair [30]. In some studies, the use of the promoter Tie2 to detect leukocytes that have become endothelial cells has rendered post hoc interpretation problematic since Tie2 labels both leukocytes and endothelial cells [39]. Therefore the claims that CEPs become endothelial cells may be over stated. In these current studies we consistently found that almost all the recruited human HSCs retained the common leukocyte marker CD45, which is consistent with other published data [11]. We used the endothelial marker CD31 to identify human endothelial cells, but CD31 also labels B cells and monocyte/macrophages [40], and proved to be non-specific for endothelial cells in these studies. However, occasional cells were identified within the capillary wall with endothelial morphology, expression of CD31, von Willebrand Factor (vWF) and absence of CD45 [41], consistent with human endothelial cells. In our initial characterization of purified HSCs there was a minor population of purified CECs (<0.05%) [38,42], and a small population of CEPs. In the post IRI kidney, the majority of human HSCs acquired CEP markers. It is possible therefore that these occasional human endothelial cells derive from cell fusion of CEPs with endothelial cells, incorporation of rare CECs, or transdifferentiation or CEPs. Certainly, appearance of human endothelial cells was not a significant mechanism of kidney PTC regeneration. Rather, our studies indicate that HSCs and the HSCs recruited to the repairing kidney that have CEP markers are capable of secretion of angiogenic factors including VEGF-A, HGF, ANG-1, IL-8, IGF-1 and FGF-2 as has been reported in other studies [18, 24, 43]. All of these cytokines are recognized as potent angiogenic factors and can promote kidney repair by increased angiogenesis [44-46]. Combined with the intravascular and perivascular locale of HSCs in the kidney we propose that the major mechanism of both survival and kidney repair by HSCs is PTC regeneration by paracrine mechanisms [24].

In conclusion, we demonstrate here that systematically administered peripheral blood mobilized human HSCs reduce mortality and promote rapid renal repair and regeneration of the kidney by paracrine mechanisms directed at peritubular capillaries. These findings support human HSCs as a promising therapeutic strategy for treatment of acute kidney diseases, and in the prevention of chronic kidney diseases.

### REFERENCES

### References Cited In Example 1

1. Duffield JS, Park KM, Hsiao LL, et al. Restoration of tubular epithelial cells during repair of the postischemic kidney occurs independently of bone marrow-derived stem cells. J Clin Invest. Jul 2005; 115(7):1743-1755.
2. Lin SL, Kisseleva T, Brenner DA, et al. Pericytes and perivascular fibroblasts are the primary source of collagen-producing cells in obstructive fibrosis of the kidney. Am JPathol. Dec 2008;173(6):1617-1627.
3. Kang DH, Joly AH, Oh SW, et al. Impaired angiogenesis in the remnant kidney model: I. Potential role of vascular endothelial growth factor and thrombospondin-1. JAm SocNephrol. Jul 2001;12(7):1434-1447.
4. Tei K, Matsumoto T, Mifune Y, et al. Administrations of peripheral blood CD34- positive cells contribute to medial collateral ligament healing via vasculogenesis. Stem Cells. Mar 2008;26(3):819-830.

### References Cited Herein Elsewhere

1. Meguid El Nahas A, Bello AK. Chronic kidney disease: the global challenge. Lancet. Jan 22-28 2005;365(9456):331-340.
2. Thadhani R, Pascual M, Bonventre JV. Acute renal failure. N Engl J Med. May 30, 1996;334(22):1448-1460.
3. Ali T, Khan I, Simpson W, et al. Incidence and outcomes in acute kidney injury: a comprehensive population-based study. JAm Soc Nephrol. Apr 2007;18(4):1292-1298.
4. Weiner DE, Tighiouart H, Amin MG, et al. Chronic kidney disease as a risk factor for cardiovascular disease and all-cause mortality: a pooled analysis of community-based studies. JAm Soc Nephrol. May 2004;15(5):1307-1315.
5. Sagrinati C, Ronconi E, Lazzeri E, et al. Stem-cell approaches for kidney repair: choosing the right cells. Trends Mol Med. Jul 2008;14(7):277-285.
6. Togel F, Weiss K, Yang Y, et al. Vasculotropic, paracrine actions of infused mesenchymal stem cells are important to the recovery from acute kidney injury. Am JPhysiol Renal Physiol. May 2007;292(5):F1626-1635.
7. Li B, Morioka T, Uchiyama M, et al. Bone marrow cell infusion ameliorates progressive glomerulosclerosis in an experimental rat model. Kidney Int. Jan 2006;69(2):323-330.
8. Van Huyen JP, Smadja DM, Bruneval P, et al. Bone marrow-derived mononuclear cell therapy induces distal angiogenesis after local injection in critical leg ischemia. Mod Pathol. Jul 2008;21(7):837-846.
9. Bi B, Schmitt R, Israilova M, et al. Stromal cells protect against acute tubular injury via an endocrine effect. J Am Soc Nephrol. Sep 2007;18(9):2486-2496.
10. Imberti B, Morigi M, Tomasoni S, et al. Insulin-like growth factor-1 sustains stem cell mediated renal repair. JAm Soc Nephrol. Nov 2007;18(11):2921-2928.
11. Dekel B, Shezen E, Even-Tov-Friedman S, et al. Transplantation of human hematopoietic stem cells into ischemic and growing kidneys suggests a role in vasculogenesis but not tubulogenesis. Stem Cells. May 2006;24(5):1185-1193.
12. Basile DP. Challenges of targeting vascular stability in acute kidney injury. Kidney Int. Aug 2008;74(3):257-258.
13. Basile DP. The endothelial cell in ischemic acute kidney injury: implications for acute and chronic function. Kidney Int. Jul 2007;72(2):151-156.
14. Basile DP, Donohoe D, Roethe K, et al. Renal ischemic injury results in permanent damage to peritubular capillaries and influences long-term function. Am JPhysiol Renal Physiol. Nov 2001;281(5):F887-899.
15. Yuan HT, Li XZ, Pitera JE, et al. Peritubular capillary loss after mouse acute nephrotoxicity correlates with down-regulation of vascular endothelial growth factor-A and hypoxia-inducible factor-1 alpha. Am J Pathol. Dec 2003;163(6):2289-2301.
16. Taguchi A, Soma T, Tanaka H, et al. Administration of CD34+ cells after stroke enhances neurogenesis via angiogenesis in a mouse model. J Clin Invest. Aug 2004;114(3):330-338.
17. Tei K, Matsumoto T, Mifune Y, et al. Administrations of peripheral blood CD34-positive cells contribute to medial collateral ligament healing via vasculogenesis. Stem Cells. Mar 2008;26(3):819-830.
18. Rafii S, Lyden D. Therapeutic stem and progenitor cell transplantation for organ vascularization and regeneration. Nat Med. Jun 2003;9(6):702-712.
19. Bertolini F, Shaked Y, Mancuso P, et al. The multifaceted circulating endothelial cell in cancer: towards marker and target identification. Nat Rev Cancer. Nov 2006;6(11):835-845.
20. Furuichi K, Gao JL, Murphy PM. Chemokine receptor CX3CR1 regulates renal interstitial fibrosis after ischemia-reperfusion injury. Am J Pathol. Aug 2006;169(2):372-387.
21. Kim J, Seok YM, Jung KJ, et al. Reactive oxygen species/oxidative stress contributes to progression of kidney fibrosis following transient ischemic injury in mice. Am J Physiol Renal Physiol. May 20 2009.
22. Ishani A, Xue JL, Himmelfarb J, et al. Acute kidney injury increases risk of ESRD among elderly. JAm Soc Nephrol. Jan 2009;20(1):223-228.
23. Bagshaw SM. Short-and long-term survival after acute kidney injury. Nephrol Dial Transplant. Jul 2008;23(7):2126-2128.
24. Matsumoto T, Kawamoto A, Kuroda R, et al. Therapeutic potential of vasculogenesis and osteogenesis promoted by peripheral blood CD34-positive cells for functional bone healing. Am J Pathol. Oct 2006;169(4):1440-1457.
25. Weisel KC, Moore MA, Kanz L, et al. Extended in vitro expansion of adult, mobilized CD34+ cells without significant cell senescence using a stromal cell coculture system with single cytokine support. Stem Cells Dev. Mar 2009; 18(2):229-234.
26. Gaia S, Smedile A, Omede P, et al. Feasibility and safety of G-CSF administration to induce bone marrow-derived cells mobilization in patients with end stage liver disease. J Hepatol. Jul 2006;45(1):13-19.
27. 27. Van Epps DE, Bender J, Lee W, et al. Harvesting, characterization, and culture of CD34+ cells from human bone marrow, peripheral blood, and cord blood. Blood Cells. 1994;20(2-3):411-423.
28. Kollet O, Shivtiel S, Chen YQ, et al. HGF, SDF-1, and MMP-9 are involved in stress-induced human CD34+ stem cell recruitment to the liver. J Clin Invest. Jul 2003;112(2):160-169.
29. Ceradini DJ, Kulkami AR, Callaghan MJ, et al. Progenitor cell trafficking is regulated by hypoxic gradients through HIF-1 induction of SDF-1. Nat Med. Aug 2004;10(8):858-864.
30. Duffield JS, Park KM, Hsiao LL, et al. Restoration of tubular epithelial cells during repair of the postischemic kidney occurs independently of bone marrow-derived stem cells. J Clin Invest. Jul 2005; 115(7): 1743-1755.
31. Lombardi D, Gordon KL, Polinsky P, et al. Salt-sensitive hypertension develops after short-term exposure to Angiotensin II. Hypertension. Apr 1999;33(4):1013-1019.
32. Carmeliet P. Angiogenesis in life, disease and medicine. Nature. Dec 15 2005;438(7070):932-936.
33. Freedman SB, Isner JM. Therapeutic angiogenesis for coronary artery disease. Ann Intern Med. Jan 1 2002;136(1):54-71.
34. Lin SL, Kisseleva T, Brenner DA, et al. Pericytes and perivascular fibroblasts are the primary source of collagen-producing cells in obstructive fibrosis of the kidney. Am J Patho/. Dec 2008;173(6):1617-1627.
35. Thomas SE, Anderson S, Gordon KL, et al. Tubulointerstitial disease in aging: evidence for underlying peritubular capillary damage, a potential role for renal ischemia. JAm Soc Nephrol. Feb 1998;9(2):231-242.
36. Ascon DB, Lopez-Briones S, Liu M, et al. Phenotypic and functional characterization of kidney-infiltrating lymphocytes in renal ischemia reperfusion injury. J Immunol. Sep 1 2006;177(5):3380-3387.
37. Duffield JS, Tipping PG, Kipari T, et al. Conditional ablation of macrophages halts progression of crescentic glomerulonephritis. Am J Pathol. Nov 2005;167(5):1207-1219.
38. Asahara T, Kawamoto A. Endothelial progenitor cells for postnatal vasculogenesis. Am JPhysiol Cell Physiol. Sep 2004;287(3):C572-579.
39. Lewis CE, De Palma M, Naldini L. Tie2-expressing monocytes and tumor angiogenesis: regulation by hypoxia and angiopoietin-2. Cancer Res. Sep 15, 2007;67(18):8429-8432.
40. Newman PJ. The biology of PECAM-1. J Clin Invest. Dec 1 1997;100(11 Suppl):S25-29.
41. Yeh ET, Zhang S, Wu HD, et al. Transdifferentiation of human peripheral blood CD34+-enriched cell population into cardiomyocytes, endothelial cells, and smooth muscle cells in vivo. Circulation. Oct 28 2003; 108(17):2070-2073.
42. Matsumoto T, Kuroda R, Mifune Y, et al. Circulating endothelial/skeletal progenitor cells for bone regeneration and healing. Bone. Sep 2008;43(3):434-439.
43. Hattori K, Dias S, Heissig B, et al. Vascular endothelial growth factor and angiopoietin-1 stimulate postnatal hematopoiesis by recruitment of vasculogenic and hematopoietic stem cells. J Exp Med. May 7 2001;193(9):1005-1014.
44. Losordo DW, Dimmeler S. Therapeutic angiogenesis and vasculogenesis for ischemic disease. Part I: angiogenic cytokines. Circulation. Jun 2004;109(21):2487-2491.
45. Brindle NP, Saharinen P, Alitalo K. Signaling and functions of angiopoietin-1 in vascular protection. Circ Res. Apr 28, 2006;98(8):1014-1023.
46. Haider H, Jiang S, Idris NM, et al. IGF-1-overexpressing mesenchymal stem cells accelerate bone marrow stem cell mobilization via paracrine activation of SDF-1alpha/CXCR4 signaling to promote myocardial repair. Circ Res. Nov 21, 2008;103(11):1300-1308.

The foregoing summary is not intended to define every aspect of the invention, and additional aspects are described in other sections, such as the Detailed Description. The entire document is intended to be related as a unified disclosure, and it should be understood that all combinations of features described herein are contemplated, even if the combination of features are not found together in the same sentence, or paragraph, or section of this document.

## Claims

1. An amount of hematopoietic stem cells (HSCs) for use in treating a kidney injury in a patient or preventing a renal disease or renal medical condition in a patient comprising a kidney injury, or increasing survival of a patient comprising a kidney injury, wherein the amount of HSCs is to be administered at least 20 hours post injury and before 7 days post injury, wherein the amount is effective to treat the kidney injury in the patient, to prevent the renal disease or renal medical condition in the patient, or to increase survival of the patient respectively; and wherein the HSCs are part of a heterogeneous cell population of which at least 70% of the population are CD 34+ cells.

2. The amount of HSCs for use according to claim 1, wherein the renal disease or renal medical condition is selected from a group consisting of: acute renal failure, chronic kidney disease, and interstitial fibrosis.

3. The amount of HSCs for use according to any of claims 1 to 2, wherein the kidney injury causes peritubular capillary loss in a kidney of the patient.

4. The amount of HSCs for use according to any of claims 1 to 3, wherein the acute kidney injury is caused by one or more of: ischemia, a toxin, use of an angiotensin-converting enzyme inhibitor (ACEI) or angiotensin II receptor blocker, a blood transfusion reaction, an injury or trauma to muscle, surgery, shock, and hypotension in the patient.

5. The amount of HSCs for use according to any of claims 1 to 4, wherein the kidney injury is renal ischemia reperfusion injury.

6. The amount of HSCs for use according to any of claims 1 to 5, wherein the HSCs are to be administered at least 22 hours post injury and before 4 days post injury, optionally wherein the HSCs are for administering at approximately 24 hours post injury.

7. The amount of HSCs for use according to any of the preceding claims, further comprising a second amount of HSCs for administration to the patient.

8. The amount of HSCs for use according to claim 7, wherein the second administration of HSCs is to be administered at least 12 hours after the first administration, optionally wherein the second administration of HSCs is for administration at least 24 hours after the first administration.

9. The amount of HSCs for use according to any of the preceding claims, wherein the HSCs are part of a heterogeneous cell population of which at least 75% of the cells are CD34+ HSCs, optionally wherein more than 75% of the cells are CD34+ HSCs.

10. The amount of HSCs for use according to any of the preceding claims, wherein the HSCs are mobilized bone marrow cells isolated from peripheral blood of a donor, optionally wherein the donor is the patient and the HSCs are autologous to the patient.

11. The amount of HSCs for use according to any of claims 1 to 10, wherein at least 2.5 x 10⁶ HSCs are administered to the patient.

12. The amount of HSCs for use according to any of the foregoing claims, wherein the HSCs are administered parenterally.

## Patentansprüche

1. Eine Menge von hämatopoetischen Stammzellen (*hematopoietic stem cells;* HSCs) zur Verwendung in der Behandlung einer Nierenverletzung in einem Patienten, oder der Vorbeugung einer Nierenerkrankung oder eines medizinischen Zustands der Nieren in einem Patienten mit einer Nierenverletzung, oder der Verbesserung des Überlebens eines Patienten mit einer Nierenverletzung, wobei die Menge von HSCs mindestens 20 Stunden nach der Verletzung und vor 7 Tagen nach der Verletzung verabreicht werden soll, wobei die Menge jeweils wirksam ist, die Nierenverletzung in dem Patienten zu behandeln, der Nierenerkrankung oder dem medizinischen Zustand der Nieren in dem Patienten vorzubeugen, oder das Überleben des Patienten zu verbessern, und wobei die HSCs Teil einer heterogenen Zellpopulation sind, von der mindestens 70% der Population CD 34⁺-Zellen sind.

2. Die Menge von HSCs zur Verwendung nach Anspruch 1, wobei die Nierenerkrankung oder der medizinische Zustand der Nieren ausgewählt ist aus der Gruppe, bestehend aus: akutem Nierenversagen, chronischer Nierenerkrankung und interstitieller Fibrose.

3. Die Menge von HSCs zur Verwendung nach einem der Ansprüche 1 und 2, wobei die Nierenverletzung einen Verlust peritubulärer Kapillaren in einer Niere der Patienten verursacht.

4. Die Menge von HSCs zur Verwendung nach einem der Ansprüche 1 bis 3, wobei die akute Nierenverletzung verursacht wird durch eines oder mehrere aus: Ischämie, einem Toxin, Verwendung eines Angiotensin-konvertierenden Enzym-Hemmers (ACEI-Hemmers) oder Angiotensin II-Rezeptor-Blockers, einer Reaktion auf eine Bluttransfusion, einer Verletzung oder einem Trauma eines Muskels, einer Operation, Schock, und Hypotonie in dem Patienten.

5. Die Menge von HSCs zur Verwendung nach einem der Ansprüche 1 bis 4, wobei die Nierenverletzung Reperfusionsschaden bei renaler Ischämie ist.

6. Die Menge von HSCs zur Verwendung nach einem der Ansprüche 1 bis 5, wobei die HSCs mindestens 22 Stunden nach der Verletzung und vor 4 Tagen nach der Verletzung verabreicht werden sollen, gegebenenfalls wobei die HSCs für eine Verabreichung etwa 24 Stunden nach der Verletzung vorgesehen sind.

7. Die Menge von HSCs zur Verwendung nach einem der vorhergehenden Ansprüche, weiter umfassend eine zweite Menge von HSCs für die Verabreichung an den Patienten.

8. Die Menge von HSCs zur Verwendung nach Anspruch 7, wobei die zweite Verabreichung von HSCs mindestens 12 Stunden nach der ersten Verabreichung verabreicht werden soll, gegebenenfalls wobei die zweite Verabreichung von HSCs für eine Verabreichung mindestens 24 Stunden nach der ersten Verabreichung vorgesehen ist.

9. Die Menge von HSCs zur Verwendung nach einem der vorhergehenden Ansprüche, wobei die HSCs Teil einer heterogenen Zellpopulation sind, von der mindestens 75% der Zellen CD34⁺-HSCs sind, gegebenenfalls wobei mehr als 75% der Zellen CD34⁺-HSCs sind.

10. Die Menge von HSCs zur Verwendung nach einem der vorhergehenden Ansprüche, wobei die HSCs mobilisierte Knochenmarkszellen sind, die aus peripherem Blut eines Spenders isoliert sind, gegebenenfalls wobei der Spender der Patient ist und die HSCs zu dem Patienten autolog sind.

11. Die Menge von HSCs zur Verwendung nach einem der Ansprüche 1 bis 10, wobei mindestens 2,5 x 10⁶ HSCs an den Patienten verabreicht werden.

12. Die Menge von HSCs zur Verwendung nach einem der vorhergehenden Ansprüche, wobei die HSCs parenteral verabreicht werden.

## Revendications

1. Quantité de cellules souches hématopoïétiques (CSH) pour une utilisation dans le traitement d'une lésion rénale chez un patient ou pour la prévention d'une maladie rénale ou d'une affection médicale rénale chez un patient comprenant une lésion rénale, ou pour l'augmentation de la survie d'un patient comprenant une lésion rénale, où la quantité de CSH doit être administrée au moins 20 heures après la lésion et avant 7 jours après la lésion, où la quantité est efficace, respectivement, pour traiter la lésion rénale chez le patient, pour prévenir la maladie rénale ou l'affection médicale rénale chez le patient, ou pour augmenter la survie du patient ; et où les CSH font partie d'une population cellulaire hétérogène dont au moins 70% de la population sont des cellules CD34+.

2. Quantité de CSH pour une utilisation selon la revendication 1, où la maladie rénale ou l'affection médicale rénale est choisie dans un groupe constitué de : insuffisance rénale aiguë, maladie rénale chronique et fibrose interstitielle.

3. Quantité de CSH pour une utilisation selon l'une quelconque des revendications 1 ou 2, où la lésion rénale provoque une perte des capillaires péritubulaires dans un rein du patient.

4. Quantité de CSH pour une utilisation selon l'une quelconque des revendications 1 à 3, où la lésion rénale aiguë est provoquée par un(e) ou plusieurs de : une ischémie, une toxine, l'utilisation d'un inhibiteur de l'enzyme de conversion de l'angiotensine (IECA) ou d'un bloqueur du récepteur de l'angiotensine II, une réaction de transfusion sanguine, une lésion ou un traumatisme d'un muscle, une chirurgie, un choc et une hypotension chez le patient.

5. Quantité de CSH pour une utilisation selon l'une quelconque des revendications 1 à 4, où la lésion rénale est une lésion d'ischémie reperfusion rénale.

6. Quantité de CSH pour une utilisation selon l'une quelconque des revendications 1 à 5, où les CSH doivent être administrées au moins 22 heures après la lésion et avant 4 jours après la lésion, éventuellement où les CSH sont destinées à une administration à approximativement 24 heures après la lésion.

7. Quantité de CSH pour une utilisation selon l'une quelconque des revendications précédentes, comprenant en outre une seconde quantité de CSH pour une administration au patient.

8. Quantité de CSH pour une utilisation selon la revendication 7, où la seconde administration de CSH doit être administrée au moins 12 heures après la première administration, éventuellement où la seconde administration de CSH est destinée à une administration au moins 24 heures après la première administration.

9. Quantité de CSH pour une utilisation selon l'une quelconque des revendications précédentes, où les CSH font partie d'une population cellulaire hétérogène dont au moins 75 % des cellules sont des CSH CD34+, éventuellement où plus de 75 % des cellules sont des CSH CD34+.

10. Quantité de CSH pour une utilisation selon l'une quelconque des revendications précédentes, où les CSH sont des cellules mobilisées de la moelle osseuse isolées du sang périphérique d'un donneur, éventuellement où le donneur est le patient et les CSH sont autologues pour le patient.

11. Quantité de CSH pour une utilisation selon l'une quelconque des revendications 1 à 10, où au moins 2,5 x 10⁶ CSH sont administrées au patient.

12. Quantité de CSH pour une utilisation selon l'une quelconque des revendications précédentes, où les CSH sont administrés par voie parentérale.
